# EUROPÄISCHE PATENTSCHRIFT

(11) **EP 1 924 694 B1**
(45) Veröffentlichungstag und Bekanntmachung des Hinweises auf die Patenterteilung: **13.10.2010**
(21) Anmeldenummer: 06793395.2
(22) Anmeldetag: 11.09.2006
(51) Int. Cl.: C12N 15/77, C12N 9/02, C07K 14/34

(54) **VERFAHREN ZUR PRODUKTION VON AMINOSÄUREN MIT MIKROORGANISMEN**
METHOD FOR PRODUCING AMINO ACIDS USING MICRO-ORGANISMS
PROCEDE DE PRODUCTION D'ACIDES AMINES PAR DES MICRO-ORGANISMES

(30) Priorität: 15.09.2005 DE 102005043979
(43) Veröffentlichungstag der Anmeldung: 28.05.2008
(73) Patentinhaber: Forschungszentrum Jülich GmbH, 52425 Jülich (DE)
(72) Erfinder: NIEBISCH, Axel, 1020 Wien (AT); BOTT, Michael, 52428 Jülich (DE)
(74) Vertreter: Fitzner, Uwe
(86) Internationale Anmeldenummer: PCT/EP2006/066213
(87) Internationale Veröffentlichungsnummer: WO 2007/031479

(56) Entgegenhaltungen:
- EP-A2- 1 108 790
- KIMURA EIICHIRO: "Triggering mechanism of L-glutamate overproduction by DtsR1 in coryneform bacteria." JOURNAL OF BIOSCIENCE AND BIOENGINEERING 2002, Bd. 94, Nr. 6, 2002, Seiten 545-551, XP002415658 ISSN: 1389-1723 in der Anmeldung erwähnt
- VILLARINO A ET AL: "Proteomic Identification of M.tuberculosis Protein Kinase Substrates: PknB Recruits GarA, a FHA Domain-containing Protein, Through Activation Loop-mediated Interactions" JOURNAL OF MOLECULAR BIOLOGY, LONDON, GB, Bd. 350, Nr. 5, 29. Juli 2005 (2005-07-29), Seiten 953-963, XP004969254 ISSN: 0022-2836
- DATABASE EMBL [Online] 1. Oktober 2002 (2002-10-01), "FHA-domain-containing proteins" XP002415690 gefunden im EBI Database accession no. Q8NQJ3
- NIEBISCH A ET AL: "Corynebacterial protein kinase G controls 2-oxoglutarate dehydrogenase activity via the phosphorylation status of the OdhI protein" JOURNAL OF BIOLOGICAL CHEMISTRY 05 MAY 2006 UNITED STATES, Bd. 281, Nr. 18, 5. Mai 2006 (2006-05-05), Seiten 12300-12307, XP002415659 ISSN: 0021-9258 1083-351X
- DATABASE EMBL [Online] 1. März 2003 (2003-03-01), "Putative serine/threonine-protein kinase PknG" XP002415691 gefunden im EBI Database accession no. Q8FMB8
- LI J ET AL: "The FHA domain mediates phosphoprotein interactions." JOURNAL OF CELL SCIENCE DEC 2000, Bd. 113 Pt 23, Dezember 2000 (2000-12), Seiten 4143-4149, XP002415660 ISSN: 0021-9533

## Beschreibung

Diese Anmeldung nimmt die Priorität der DE 10 2005 043 979.9 in Anspruch.

Die Erfindung betrifft ein Verfahren zur Produktion von Aminosäuren in aminosäure-produzierenden Mikroorganismen bei dem die Aktivität der 2-Oxoglutarat-Dehydrogenase vermindert wird durch ein Protein (auch als sog. Transmitter-Protein bezeichnet), **dadurch gekennzeichnet, dass** das Protein nicht phosphoryliert ist.

### Stand der Technik

Aminosäuren sind von großem wirtschaftlichen Interesse, wobei die Verwendung von Aminosäuren vielfältig ist: bevorzugt werden sie zur Verbesserung der Qualität von Grundnahrungsstoffen und Futtermitteln eingesetzt. Deshalb sind sie sind von großer wirtschaftlicher Bedeutung; ihr Bedarf nimmt stetig zu. L-Lysin wird z.B.wie auch L-Threonin, L-Methionin und L-Tryptophan als Futtermittelzusatz benötigt, L-Glutamat als Gewürzzusatz, L-Isoleucin und L-Tyrosin in der pharmazeutischen Industrie, L-Arginin und L-Isoleucin als Medikament oder L-Glutamat, L-Aspartat und L-Phenylalanin als Ausgangssubstanz zur Synthese von Feinchemikalien.

Eine bevorzugte Methode zur Herstellung dieser verschiedensten Aminosäuren ist die biotechnologische Herstellung mittels Mikroorganismen; denn auf diese Weise wird direkt die biologisch wirksame und optisch aktive Form der jeweiligen Aminosäure erhalten, und es können einfache und preisgünstige Rohstoffe eingesetzt werden. Zur Zeit werden mehr als 1,5 x 10⁶ Tonnen Aminosäuren mikrobiell produziert. Es ist bekannt, daß Aminosäuren durch Fermentation von Stämmen coryneformer Bakterien, insbesondere *Corynebacterium glutamicum,* hergestellt werden. Wegen der großen Bedeutung wird ständig an Verbesserungen der Herstellverfahren gearbeitet. Verfahrensverbesserungen können fermentationstechnische Maßnahmen wie z. B. Rührung und Versorgung mit Sauerstoff, oder die Zusammensetzung der Nährmedien wie z. B. die Zuckerkonzentration während der Fermentation, oder die Aufarbeitung zur Produktform durch z.B. Ionenaustauschchromatographie oder die intrinsischen Leistungseigenschaften des Mikroorganismus selbst sein.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Metabolite sind und L-Aminosäuren produzieren.

Seit einigen Jahren werden ebenfalls Methoden der rekombinanten DNA-Technik zur Stammverbesserung Aminosäure-produzierender Stämme von *Corynebacterium* eingesetzt, indem man einzelne Aminosäure-Biosynthesewege amplifiziert und die Auswirkung auf die Aminosäureproduktion untersucht. Übersichtsartikel hierzu findet man unter anderem bei Kinoshita ("Glutamic acid bacteria", in: Biology of Industrial Microorganisms, Demain and Solomon (Eds.), Benjamin Cummings, London, UK, 1985, 115-143), Hilliger (BioTec 2, 40-44 (1991)), Eggeling (Amino Acids 6: 261-272 (1994)), Jetten und Sinskey (Critical Reviews in Biotechnology 15, 73-103 (1995)), und Sahm et al. (Annals of the New York Academy of Sciences 782, 25-39 (1996)).

Bakterien produzieren Aminosäuren normalerweise nur in der zum Wachstum benötigten Menge, so daß also keine überschüssigen Aminosäuren gebildet und ausgeschieden werden. Dies ist darin begründet, daß in der Zelle die Biosynthese der Aminosäuren in vielfacher Weise kontrolliert wird. Folglich sind bereits verschiedenste Verfahren bekannt, um die Produktbildung durch Ausschalten der Kontrollmechanismen zu steigern. Bei diesen Prozessen werden z.B. Aminosäureanaloga eingesetzt, um die effektive Regulation der Biosynthese auszuschalten. So ist beispielsweise ein Verfahren beschrieben, bei dem *Corynebacterium*-Stämme benutzt werden, die gegen L-Tyrosin- und L-Phenylalaninanaloga resistent sind (JP51019037 und JP53039517). Ebenso sind Verfahren beschrieben, bei denen gegenüber L-Lysin- oder auch L-Theoninanaloga resistente Bakterien eingesetzt werden, um die Kontrollmechanismen zu überwinden (EP 0 205 849, GB 2 152 509).

Weiterhin sind auch durch rekombinante DNA-Techniken konstruierte Mikroorgansimen bekannt, bei denen ebenfalls die Regulation der Biosynthese aufgehoben ist, indem die Gene, die für die nicht mehr feedback-inhibierbaren Schlüsselenzyme kodieren, kloniert und exprimiert werden. So ist z.B. ein rekombinantes, L-Lysin produzierendes Bakterium mit plasmid-kodierter, feedback-resistenter Aspartatkinase bekannt (EP 0 381 527). Ebenso ist ein rekombinantes, L-Phenylalanin produzierendes Bakterium mit feedback-resistenter Prephenatdehydrogenase beschrieben (JP 123475/1986, EP 0 488 424).

Darüber hinaus wurden auch durch Überexpression von Genen, die nicht für feedback-sensitive Enzyme der Aminosäuresynthese kodieren, erhöhte Aminosäureausbeuten erreicht. So wird z.B. die Lysinbildung durch erhöhte Synthese der Dihydrodipicolinatsynthase verbessert (EP 0 197 335). Ebenso wird durch erhöhte Synthese der Threonindehydratase eine verbesserte Isoleucinbildung erreicht (EP 0 436 886).

Ferner ist die fermentative Herstellung von L-Serin aus Methanol und Glycin mit Hilfe methylotropher Bakterien, beispielsweise der Gattung *Hyphomicrobium* bei Izumi Y, Yoshida T, Miyazaki SS, Mitsunaga T, Ohshiro T, Shiamo M, Miyata A und Tanabe T (1993) Applied Microbiology and Biotechnology, 39: 427-432 beschrieben.

Weitere Versuche zur Erhöhung der Aminosäureproduktion zielen auf eine verbesserte Bereitstellung der zellulären Primärmetabolite des Zentralstoffwechsels. So ist bekannt, daß die durch rekombinante Techniken erreichte Überexpression der Transketolase eine verbesserte Produktbildung von L-Tryptophan, L-Tyrosin oder L-Phenylalanin ermöglicht (EP 0 600 463).

Weiterhin führt die Reduktion der Phosphoenolpyruvat-Carboxylase-Aktivität in *Corynebacterium* zu verbesserter Bildung aromatischer Aminosäuren (EP 0 331 145), wohingegen die Erhöhung der Phosphoenolpyruvat-Carboxylase-Aktivität in *Corynebacterium* zu erhöhter Ausscheidung von Aminosäuren der Aspartatfamilie führte (EP 0 358 940).

*Corynebacterium glutamicum* wird industriell auch zur Herstellung von L-Aminosäuren der Glutamatfamilie eingesetzt, inbesondere von L-Glutamat. Aus der WO 99/18228A2 ist bekannt, daß nach Erhöhung der Pyruvat-Carboxylase-Aktivität durch genetische Veränderung des Enzyms oder nach Erhöhung der Pyruvat-Carboxylase-Genexpression die mikrobielle Herstellung von Aminosäuren der Aspartat- und/oder der Glutamatfamilie erhöht wird.

L-Glutamat wird aus dem Citrat-Zyklus-Intermediat 2-Oxoglutarat gebildet. Zwei Reaktionen konkurrieren um das 2-Oxoglutarat: Die NADPH-abhängige L-Glutamat-Dehydrogenase (GDH), die 2-Oxoglutarat mit Ammonium und reduktiv zu L-Glutamat umsetzt, und der 2-Oxoglutarat-Dehydrogenase-Komplex (ODHC), der als Bestandteil des Citrat-Zyklus 2-Oxoglutarat zu Succinyl-CoA decarboxyliert und dabei NAD⁺ zu NADH reduziert. In *C. glutamicum* ist der Kₘ-Wert der GDH für 2-Oxoglutarat mit 5.7 mM (Shiio and Ozaki, J. Biochem (Tokyo) 68: 633-647(1970)) ca. 70-fach höher als der Kₘ-Wert des ODHC für 2-Oxoglutarat, der bei 80 µM liegt (Shiio and Ujigawatakeda, Agric. Biol. Chem. 44: 1897-1904 (1980)). Neuere Untersuchungen deuten darauf hin, daß die Aktivität des 2-Oxoglutarat-Dehydrogenase-Komplexes in Corynebakterien der Faktor mit dem grössten Einfluss auf die Glutamat-Produktion darstellt (Shimizu et al., Bioproc. Biosys. Eng. 25: 291-298 (2003)), da eine Erhöhung der GDH-Aktivität keine Verschiebung zugunsten der Glutamatsynthese bewirkt.

Der Einfluß des ODH-Komplexes wird bestätigt durch Hinweise aus der Literatur, daß die Aktivität dieses Enzyms unter Bedingungen, die zur GlutamatAusscheidung führen, z.B. Biotin-Limitierung, Zugabe von Detergenz, oder Zugabe von Penicillin, erniedrigt ist (Kawahara et al., Biosci. Biotechnol. Biochem. 61: 1109-1112 (1997)). Unter den gleichen Bedingungen ist die GDH-Aktivität unverändert. Auch bei Induktion der Glutamat-Bildung durch einen temperatur-sensitiven *C. glutamicum-Stamm* konnte eine erniedrigte ODHC-Aktivität gezeigt werden (Uy et al., Bioproc. Biosyst. Eng. 27: 153-162 (2005)).

Weiterhin beschreibt Kimura (J. Biosc. Bioeng., Vol. 94, No. 6, 545-551, 2002), daß die Aktivität des ODHC bei Deletion des *dtsR1*-Gens in *C. glutamicum* um 30% geringer ist als die des Parentalstammes (Kimura, J. Bioscience Bioeng. 94: 545-551 (2002)). Die *dtsR1*-Deletionsmutante ist auxotroph für Ölsäure und Ölsäureester und exkretiert auch bei Biotin-Überschuss signifikante Mengen an L-Glutamat (Kimura et al., Biochem. Biophys. Res. Comm. 234: 157-161 (1997)).

Bisher konnte eine gezielte Veränderung der Aktivität des 2-Oxoglutarat-Dehydrogenase-Komplexes und der Glutamat-Dehydrogenase nur durch Deletion oder veränderte Expression der Gene *gdh* (Börmann et al., Mol. Microbiol. 6: 317-326 (1992)); Börmann-EI Kholy et al., Appl. Environm. Microbiol. 59: 2329-2331 (1993)) und *odhA* (kodiert E1o von ODHC; (Usuda et al., Microbiology 142: 3347-3354 (1996)) erreicht werden.

Der 2-Oxoglutarat-Dehydrogenase-Komplex (ODHC) besteht in den meisten Organismen aus drei verschiedenen Proteinen mit unterschiedlichen enzymatischen Aktivitäten, nämlich der 2-Oxoglutarat-Dehydrogenase (E1o), der Dihydrolipoamid-S-Succinyltransferase (E2) und der Dihydrolipoamid-Dehydrogenase (E3). Das Elo-Protein (kodiert durch das Gen *odhA*) von Corynebakterien besitzt einen ungewöhnlichen Aufbau, da es im aminoterminalen Bereich eine Region besitzt, die homolog ist zu dem carboxyterminalen Bereich der E2-Untereinheit (Usuda et al., Microbiology 142: 3347-3354 (1996)).

Cowley et al. (Mol. Microbiol. 52: 1691-1702 (2004)) haben gezeigt, daß in *Mycobacterium tuberculosis* H37Rv durch Deletion des *pknG*-Gens die Summe der zellulären Konzentrationen von Glutamat und Glutamin im Vergleich zum entsprechenden Parental-Stamm um den Faktor 2,64 erhöht wird. Über die intrazelluläre Konzentration der einzelnen Aminosäure, Glutamat und Glutamin, werden jedoch keine Aussagen gemacht. Das *pknG*-Gen kodiert für eine Serin/Threonin-Proteinkinase, dessen zelluläre Funktion bisher nicht beschrieben wurde. Lediglich die Bedeutung des PknG-Proteins für das Zellwachstum wurde erkannt. Eine *M. tuberculosis* H37Rv-Mutante ohne das pknGGen erreichte unter den bei Cowley et al. (Mol. Microbiol. 52: 1691-1702 (2004)) angegebenen Wachstumsbedingungen eine Zelldichte die, bestimmt über die optische Dichte bei 600 nm, maximal nur halb so hoch war wie die Zelldichte des Parentalstammes.

### Aufgabe der Erfindung

Aufgabe der vorliegenden Erfindung war es weitere Möglichkeiten zur Erhöhung der Ausscheidung von Aminosäuren-, besonders von Glutamat und Glutamin, aufzuweisen die effizienter sind und die Nachteile des Standes der Technik nicht mehr aufweisen.

### Beschreibung der Erfindung

Diese Aufgabe wird durch ein Verfahren zur Produktion von Aminosäuren in aminosäure-produzierenden Mikroorganismen gelöst, bei dem die Aktivität des 2-Oxoglutarat-Dehydrogenase-Komplexes durch ein Protein vermindert wird, **dadurch gekennzeichnet, dass** das Protein nicht phosphoryliert ist und durch eine Nukleinsäuresequenz codiert wird umfassend
a) eine Nukleinsäuresequenz mit der in SEQ ID NO:1 dargestellten Nukleinsäuresequenz aus C. glutamicum; oder
b) eine Nukleinsäuresequenz, die sich aufgrund des degenerierten genetischen Codes durch Rückübersetzung aus der in SEQ ID NO:2 aus C. glutamicum dargestellten Aminosäuresequenz ableiten lässt; oder
c) eine Nukleinsäuresequenz, welche eine Identität mit der SEQ ID NO:1 aus C. glutamicum von mindestens 50% aufweist, und die Expression eines Proteins mit den gleichen Eigenschaften wie die des Proteins aus C. glutamicum bewirkt, wobei das Protein die Aktivität des 2-Oxoglutarat-Dehydrogenase-Komplexes inhibiert, aber nicht die Expression der für den 2-Oxoglutarat-Dehydrogenase-Komplex kodierenden Gene beeinflußt..

Der Begriff "Aktivität" beschreibt die Fähigkeit eines Enzyms, ein Substrat in ein Produkt umzuwandeln. Die Aktivität kann in einem sogenannten Aktivitätstest über die Zunahme des Produktes, die Abnahme des Substrates (oder Eduktes) oder die Abnahme eines spezifischen Cofaktors oder über eine Kombination aus mindestens zwei der vorstehend genannten Parameter in Abhängigkeit von der Zeit bestimmt werden.

Als Aktivität des 2-Oxoglutarat-Dehydrogenase-Komplexes ist erfindungsgemäß die katalytische Umsetzung von 2-Oxoglutarat, Coenzym A und NAD⁺ zu Succinyl-CoA, CO₂, NADH und **H⁺** zu verstehen.

Die Erhöhung oder Erniedrigung der Aktivität, der Produktion oder der Konzentration, Zu- und Abnahme von Stoffen, Produkten, Edukten oder Substraten bezieht sich erfindungsgemäß auf den Vergleich mit dem Parentalstamm in einem Vergleichsexperiment unter identischen Bedingungen, der die erfindungsgemäße Verringerung der 2-Oxoglutarat-Dehydrogenase - Aktivität nicht aufweist.

Als Parentalstamm wird der Stamm von Mikroorganismen bezeichnet, der als Ausgangstamm dient und an dem die Änderungen vollzogen werden um die erfindungsgemäße Verringerung der 2-Oxogluturat-Dehydrogenase - Aktivität zu erzielen.

Wird die Aktivität des ODHC herabgesetzt, so wird das Substrat 2-Oxoglutarat primär durch die Glutamat-Dehydrogenase (GDH) mit Ammonium, NADPH und H⁺ zu L-Glutamat und NADP⁺ umgesetzt und dadurch der zelluläre Gehalt an Glutamat erhöht. Da L-Glutamat der wichtigste Amino-Donor bei der Synthese von L-Aminosäuren aus den entsprechenden 2-Keto-Verbindungen ist, wird durch die Erhöhung der Glutamatkonzentration auch die Produktion weiterer Aminosäuren als Folgeprodukte erhöht. Als Beispiel seien hier Glutamin, Aspartat, Omithin, Asparagin, Arginin, Lysin, Valin, Leucin, Isoleucin, Methionin, Cystein und/oder Prolin, genannt.

Die Mikroorganismen, die Gegenstand der vorliegenden Erfindung sind, können Aminosäuren, insbesondere L-Glutamat aus Glucose, Saccharose, Lactose, Fructose, Maltose, Melasse, Stärke, Cellulose oder aus Glycerin und Ethanol herstellen.

Als Mikroorganismen werden Stäbchenbakterien eingesetzt, bevorzugt gram-positive Stäbchenbakterien, *Corynebacteriaceae, Mycobacteriaceae, Propionibacteriacae, Streptomycetaceae* oder *Lactobacillacae,* besonders bevorzugt der Familie *Corynebacteriaceae* oder *Mycobacteriaceae* oder *Streptomycetaceae.*

Es kann sich um Vertreter coryneformer Bakterien wie zum Beispiel *Nocardiaceae, Dietziaceae, Gordoniaceae, Tsukamurellaceae" Rhodococcus,* Skermania, , *Williamsiaceae,* bevorzugt *Corynebacteriaceae,* insbesondere der Gattung *Corynebacterium* handeln. Bei der Gattung *Corynebacterium* sind insbesondere die Arten *Corynebacterium glutamicum* und *Corynebacterium efficiens* zu nennen, die in der Fachwelt für ihre Fähigkeit bekannt ist, L-Aminosäuren zu produzieren (Eggeling, L. and Bott, M., Handbook of Corynebacterium glutamicum, CRC Press, Boca Raton, USA (2005)).

Geeignete Stämme der Gattung *Corynebacterium,* insbesondere der Art *Corynebacterium glutamicum,* sind zum Beispiel die bekannten Wildtypstämme
*Corynebacterium glutamicum* ATCC 13032
*Corynebacterium acetoglutamicum* ATCC 15806
*Corynebacterium acetoacidophilum* ATCC13870
*Corynebacterium melassecola* ATCC17965
*Corynebacterium thermoaminogenes* FERM BP-1539
*Brevibacterium flavum* ATCC 14067
*Brevibacterium lactofermentum* ATCC13869 und
*Brevibacterium divaricatum* ATCC 14020

Prinzipiell können in dem erfindungsgemäßen Verfahren alle zur biotechnologisch, industriellen Produktion von Aminosäuren bekannten Mikroorganismen eingesetzt werden.

Ferner sind weitere Stämme coryneforme Bakterien geeignet, wie *'Brevibacterium aminogenes', 'Brevibacterium divaricatum,' Brevibacterium ammoniagenes, 'Brevibacterium flavum,' 'Brevibacterium lactofermentum,"Brevibacterium roseum,' 'Brevibacterium saccharolyticum,' 'Brevibacterium immariophilum,' 'Corynebacterium acetoacidophilum,' Corynebacterium lilium, Corynebacterium callunae,* und *'Corynebacterium herculis.'* Weitere erfindungsgemäß einzusetztende Bakerien werden in "Handbook of Corynebacterium g/utamicum', L. Eggeling und M. Bott (eds.), CRC Press, Boca Raton, USA (2005), Kapitel 2.4 erwähnt, wie zum Beispiel *C. amycolatum, C. kroppenstedtii, C. atypicum, Turicella T. otitidis, C. callunae, C. efficiens, C. ammoniagenes, C. cystitidis, C. pilosum, C. ammoniagenes, C. amycolatum, C. xerosis, C. imitans, C. pseudodiphtheriticum, C. coyleae, C. striatum, C. fastidiosum, C. mucifaciens, C. propinquum, C. confusum, C. sundsvallense, C. thomssenii, C. glaucum, C. lipophiloflavum, C. mycetoides, C. appendecis, C. imitans, C. callunae, C. efficiens, C. flcescens, C. urealyticum, C. jeikeium, C. falsenii, C. bovis, C. variabillis, C. diphtheriae, C. vitaeruminis, C. kutscheri, C. glucuronolyticum, C. renale, C. mastitidis, C. durum, C. nigricans, C. minutissimum, C. simulans, C. terpenotabidum, C. pseudotuberculosis, C. pseudodiphtheriticum, C. coyleae.*

Die erfindungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) zum Zwecke der Produktion von Aminosäuren, kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z.B. Palmitinsäure, Stearinsäure und Linolsäure, Alkohole wie z. B. Glycerin und Ethanol und organische Säuren wie z. B. Essigsäure verwendet werden. Als Stickstoffquelle können organische Stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoff-haltige Gasmischungen wie z. B. Luft in die Kultur eingetragen. Die Temperatur der Kultur liegt normalerweise bei 20°C bis 45 °C, vorzugsweise bei 25°C bis 40 °C. Die Kultur wird solange fortgesetzt, bis sich ein Maximum an Aminosäuren, bevorzugt an Glutamat gebildet hat.

Bei der Kultivierung der oben aufgeführten Mikroorganismen kann die Glutamatausscheidung durch die dem Fachmann bekannten Methoden induziert werden, z. B. Biotin-Limitierung, Zugabe von Penicillin oder anderen Antibiotika, Zugabe von Detergenzien wie Tween-40 und Tween-60, Zugabe von Ethambutol, Ausschalten des *dtsR1*-Gens und anderer Gene der FettsäureBiosynthese oder Temperatur-Erhöhung.

Die Induzierung der Glutamatbildung bedeutet eine Erhöhung der Glutamatausscheidung im Vergleich zur Kultivierungen der Mikroorganismen ohne die oben genannten Änderungen der Bedingungen.

Die Analyse von L-Glutamat kann durch reversed-phase HochdruckFlüssigkeitschromatographie nach Derivatisierung mit *ortho*-Phtaldialdehyd durch Fluoreszensdetektion erfolgen, so wie bei Lindroth und Mopper (Anal. Chem. 51: 1667-1674 (1979)) beschrieben.

Bevorzugt wird in dem erfindungsgemäßen Verfahren die Produktion des L-Isomers der Aminosäure erhöht.

Die Bezeichnungen der Aminosäuren in ihrer protonierten Form, z. B. Glutaminsäure, in ihrer deprotonierten Form, z. B. Glutamat, und in ihrer Salzform, z. B. Natrium-Glutamat oder Natrium-Aspartat, sind äquivalent.

In einer Ausführungsform der vorliegenden Erfindung wird die Produktion der Aminosäure erhöht um mindestens Faktor 1,1; 1,2; 1,3; 1,4; 1,5; 2,0; 3,0; bevorzugt 4,0; 5,0; besonders bevorzugt 10,0 oder größer.

Wesentliches Merkmal des erfindungsgemäßen Verfahrens ist die Verminderung der Aktivität des 2-Oxoglutarat-Dehydrogenase-Komplexes durch ein Protein, welches auch als Transmitter-Protein bezeichnet wird.

Als Transmitter-Protein wird ein Protein bezeichnet, daß die Aktivität des 2-Oxoglutarat-Dehydrogenase-Komplexes inhibiert, aber nicht die Expression der für den 2-Oxoglutarat-Dehydrogenase-Komplex kodierenden Gene beeinflußt.

Das im erfindungsgemäßen Verfahren verwendete Protein wird codiert durch eine Nukleinsäuresequenz umfassend:
a) eine Nukleinsäuresequenz mit der in SEQU. ID No. 1 dargestellten Nukleinsäuresequenz; oder
b) eine Nukleinsäuresequenz, die sich aufgrund des degenerierten genetischen Codes durch Rückübersetzung aus der in SEQU. ID No. 2 dargestellten Aminosäuresequenz ableiten lässt; oder
c) eine Nukleinsäuresequenz, welche eine Identität mit der SEQ ID NO:1 von mindestens 50% aufweist.

Bevorzugt weist die Nukleinsäuresequenz c) eine Identität mit der SEQ ID NO:1 von mindestens 60%, 65%, 70%, 75%, 80%, besonders bevorzugt 85%, 90%, insbesondere 91%, 92%, 93%, 94%, 95%, 96%, 97%, 98% oder 99% auf.

Anstelle des Begriffs "Identität" kann auch der Begriff "homolog" oder "Homologie" gleichbedeutend verwendet werden.

Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren:
Gap creation penalty: 8
Gap extension penalty: 2
und folgender Parameter für Nukleinsäuren
Gap creation penalty: 50
Gap extension penalty: 3.

Bevorzugt wird die Identität zwischen zwei Nukleinsäuresequenzen oder Polypeptidsequenzen durch die Identität der Nukleinsäuresequenz/Polypeptidsequenz über die jeweils gesamte Sequenzlänge definiert, wie sie durch Vergleich mit Hilfe des Programms ***GAP*** basierend auf dem Algorithmus von Needleman, S.B. und Wunsch, C.D. (J. Mol. Biol. 48: 443-453)) berechnet wird unter Einstellung folgender Parameter für Aminosäuren Gap creation penalty: 8

Gap extension penalty: 2
und die folgenden Parameter für Nukleinsäuren
Gap creation penalty: 50
Gap extension penalty: 3.

In dem erfindungsgemäßen Verfahren können auch weitere Homologe oder funktionelle Äquivalente eingesetzt werden, welche eine Identität mit der SEQ ID NO:1 von mindestens 50% aufweisen, und die Expression eines Proteins mit den gleichen Eigenschaften wie die des Proteins aus C. glutamicum bewirkt, wobei das Protein die Aktivität des 2-Oxoglutarat-Dehydrogenase-Komplexes inhibiert, aber nicht die Expression der für den 2-Oxoglutarat-Dehydrogenase-Komplex kodierenden Gene beeinflußt.

"Funktionelle Äquivalente" beschreiben prinzipiell hier Nukleinsäuresequenzen, die unter Standardbedingungen mit einer Nukleinsäuresequenz oder Teilen einer Nukleinsäuresequenz hybridisieren und befähigt sind, die Expression eines Proteins mit den gleichen Eigenschaften wie die des Proteins Odhl aus *C. glutamicum* in einer Zelle oder einem Organismus zu bewirken.

Zur Hybridisierung werden vorteilhaft kurze Oligonukleotide mit einer Länge von etwa 10-50 bp, vorzugsweise 15-40 bp beispielsweise der konservierten oder sonstigen Bereiche, die über Vergleiche mit anderen verwandten Genen in dem Fachmann bekannter Weise ermittelt werden können, verwendet. Es können aber auch längere Fragmente der erfindungsgemäßen Nukleinsäuren mit einer Länge von 100-500 bp oder die vollständigen Sequenzen für die Hybridisierung verwendet werden. Je nach der verwendeten Nukleinsäure/Oligonukleotid, der Länge des Fragmentes oder der vollständigen Sequenz oder je nachdem welche Nukleinsäureart, d.h. DNA oder RNA, für die Hybridisierung verwendet werden, variieren diese Standardbedingungen. So liegen beispielsweise die Schmelztemperaturen für DNA:DNA-Hybride ca. 10°C niedriger als die von DNA:RNA-Hybriden gleicher Länge.

Unter Standardhybridisierungsbbedingungen sind beispielsweise je nach Nukleinsäure Temperaturen zwischen 42 und 58°C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20°C bis 65°C, bevorzugt zwischen etwa 30°C bis 45°C. Für DNA:RNA-Hybride liegen die Hybridisierungsbedingungen vorteilhaft bei 0,1 x SSC und Temperaturen zwischen etwa 30°C bis 65°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50% in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, "Current Protocols in Molecular Biology", John Wiley & Sons, New York; Hames and Higgins (eds), 1985, "Nucleic Acids Hybridization: A Practical Approach", IRL Press at Oxford University Press, Oxford; Brown (ed), 1991, Essential Molecular Biology: A Practical Approach, IRL Press at Oxford University Press, Oxford.

Unter einem funktionellen Äquivalent versteht man weiterhin auch Nukleinsäuresequenzen, die mit einer bestimmten Nukleinsäuresequenz ("ursprüngliche Nukleinsäuresequenz) bis zu einem definierten Prozentsatz homolog bzw. identisch sind und die gleiche Aktivität wie die ursprünglichen Nukleinsäuresequenzen aufweisen, ferner insbesondere auch natürliche oder künstliche Mutationen dieser Nukleinsäuresequenzen.

Wesentlich für die Funktion des Proteins Odhl ist das Aminosäure-Sequenzmotiv Glu-Thr-Thr-Ser von Odhl. Gegenstand der Erfindung sind ferner funktionelle Äquivalente von Odhl, die das Aminosäure-Sequenzmotiv Glu-Thr-Thr-Ser enthalten.

Das erfindungsgemäße Protein Odhl inhibiert die Aktivität des 2-Oxoglutarat-Dehydrogenase-Komplexes im nicht phosphoryliertem Zustand. Bevorzugt ist das Protein an den Threonin-Resten an den Positionen 14 und/oder 15, bevorzugt 14, nicht phosphoryliert.

In dieser Ausführungsform bezieht sich die Erhöhung oder Erniedrigung der Aktivität, der Produktion oder der Konzentration, Zu- und Abnahme von Stoffen, Produkten, Edukten oder Substraten erfindungsgemäß auf den Vergleich mit Parentalstamm in einem Vergleichsexperiment der ein zumindest teilweise phosphoryliertes Protein Odhl aufweist.

In dieser Ausführungsform der vorliegenden Erfindung wird die Produktion der Aminosäure erhöht um mindestens Faktor 1,1; 1,2; 1,3; 1,4; 1,5; 2,0; 3,0: bevorzugt 4,0; 5,0; besonders bevorzugt 10,0 oder größer.

In einer Ausführungsform der Erfindung wird die Phosphorylierung von Odhl durch die Inaktivierung der Proteinkinase PknG verhindert.

Bevorzugt wird das Protein nicht durch die Proteinkinase PknG phosphoryliert, welche durch eine Nukleinsäuresequenz codiert wird umfassend:
a) eine Nukleinsäuresequenz mit der in SEQU. ID No. 3 dargestellten Nukleinsäuresequenz ; oder
b) eine Nukleinsäuresequenz, die sich aufgrund des degenerierten genetischen Codes durch Rückübersetzung aus der in SEQU. ID No. 4 dargestellten Aminosäuresequenz ableiten lässt; oder
c) eine Nukleinsäuresequenz, welche eine Identität mit der SEQ ID NO:3 von mindestens 50% aufweist.

In einer bevorzugten Ausführung des erfindungsgemäßen Verfahrens wird die Aktivität der Proteinkinase PknG vermindert.

Erfindungsgemäß kann die verminderte Aktivität der Proteinkinase PknG durch Veränderungen auf transkriptionaler, translationaler und/oder posttranslationaler Ebene hervorgerufen werden. Hierunter sind beispielsweise Veränderungen regulativer Elemente, wie z. B. der Promotoraktivitäten, der Beständigkeit der gebildeten mRNAs oder Proteine gegenüber Abbau durch Nukleasen oder Proteasen oder der Inititation der Translation durch Ribosomenbindung oder Verringerung der katalytischen Enzymaktivität selbst oder durch verändertes Bindungsverhalten von Regulatoren oder andere Proteine zu verstehen. Weiterhin auch Veränderungen in der Expression durch Inaktivierung oder Modifizierung von Transkriptionsregulatoren oder RNA-Polymerase-Untereinheiten.

Darüber hinaus ist auch eine geringfügige oder gänzlich fehlende Aktivität der Proteinkinase PknG durch die Inaktivierung des *pknG-*Gens denkbar, welche erfindungsgemäß bevorzugt ist. Dies kann durch verschiedenartige, an sich bekannte gentechnische Ansätze erreicht werden, wie z. B. Insertions- oder Deletionsmutagenese oder chemische Behandlung der Zellen mit mutagenen Agenzien. Bevorzugt sind *pknG*-Deletionsmutanten. Die zuvor genannten Methoden dienen dabei lediglich der Erläuterung der Erfindung und wirken nicht limitierend.

Besonders bevorzugt wird die Aktivität der Proteinkinase PknG durch Deletion der Nukleinsäuresequenz umfassend:
a) eine Nukleinsäuresequenz mit der in SEQU. ID No. 3 dargestellten Nukleinsäuresequenz ; oder
b) eine Nukleinsäuresequenz, die sich aufgrund des degenerierten genetischen Codes durch Rückübersetzung aus der in SEQU. ID No. 4 dargestellten Aminosäuresequenz ableiten lässt; oder
c) eine Nukleinsäuresequenz, welche eine Identität mit der SEQ ID NO:3 von mindestens 50% aufweist,
vermindert.

Bevorzugt weist die Nukleinsäuresequenz c) eine Identität mit der SEQ ID NO:3 von mindestens 60%, 65%, 70%, 75%, 80%, besonders bevorzugt 85%, 90%, insbesondere 91%, 92%, 93%,94%, 95%, 96%, 97%, 98% oder 99% auf.

Anstelle des Begriffs "Identität" kann auch der Begriff "homolog" oder "Homologie" gleichbedeutend verwendet werden.

Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren:
Gap creation penalty: 8
Gap extension penalty: 2
und folgender Parameter für Nukleinsäuren
Gap creation penalty: 50
Gap extension penalty: 3.

Bevorzugt wird die Identität zwischen zwei Nukleinsäuresequenzen oder Polypeptidsequenzen durch die Identität der Nukleinsäuresequenz/Polypeptidsequenz über die jeweils gesamte Sequenzlänge definiert, wie sie durch Vergleich mit Hilfe des Programms ***GAP*** basierend auf dem Algorithmus von Needleman, S.B. und Wunsch, C.D. (J. Mol. Biol. 48: 443-453)) berechnet wird unter Einstellung folgender Parameter für Aminosäuren
Gap creation penalty: 8
Gap extension penalty: 2
und die folgenden Parameter für Nukleinsäuren
Gap creation penalty: 50
Gap extension penalty: 3.

Ein Verfahren zur Deletion von Genen, das auch zur erfindungsgemäßen Deletion des *pknG*-Gens eingesetzt werden kann, wird von Niebisch and Bott in Arch. Microbiol 175, 282-294 (2001) beschrieben.

In einer anderen bevorzugten Ausführungsform der vorliegenden Erfindung wird die Inaktivierung des *pknG*-Gens mittels dem Fachmann bekannter Verfahren erzielt, wie zum Beispiel RNA-interference, anti-sense-Technik oder Gene silencing.

Ferner kann die Inaktivierung des *pknG*-Gens durch Mutation jener Aminosäurereste von PknG erreicht werden, die für die Phosphorylierung des Odhl-Proteins notwendig sind.

In einer weiteren bevorzugten Ausführungsform der vorliegenden Erfindung wird die Odhl-vermittelte Hemmung der ODHC-Aktivität durch Mutation der Threonin-Reste 14 und/oder 15 im Odhl-Protein und Expression der mutierten Proteine in Corynebakterien erzielt, wobei gekennzeichnet, daß das Protein an den Aminosäuren Threonin an den Positionen 14 und/oder 15, bevorzugt 14 nicht phosphoryliert ist.

Der Vorteil des erfindungsgemäßen Verfahrens liegt in der deutlichen Erhöhung der Produktion von Aminosäuren, bevorzugt Glutamat, im Vergleich zu den jeweiligen Parentalstämmen. Besonders die spezifische Produktivität, das heißt die pro Zeit und Zellmasse ins Medium ausgeschiedene Menge Aminosäuren wird erhöht.

Ferner führt das erfindungsgemäße Verfahren zur einer höheren Produktivität (Raum-Zeit-Ausbeute).

Im folgenden wird die Erfindung unter Bezugnahme auf die Beispiele näher erläutert:

### Beispiele:

### 1. Deletion der Gene pknG und/oder odhI in Corynebacterium glutamicum

Zur Deletion von Genen in *Corynebakterium glutamicum* Atcc13032 wurde die bei Niebisch und Bott (Arch. Microbiol. 175: 282-294 (2001)) beschriebene Methode eingesetzt.

Für die Amplifizierung der 5'- und 3'-flankierenden Bereiche des *pknG-Gens* wurden die Oligonukleotid-Paare DpknG-1 (SEQ ID NO 5) und DpknG-2 (SEQ ID NO 6 ) (5'-Bereich) bzw. DpknG-3 (SEQ ID NO 7) und DpknG-4 (SEQ ID NO 8) (3'-Bereich) eingesetzt:
DpknG-1: 5'-GACGTCGACGATCACCGACGAACGCGC-3' ; SEQ ID NO 5
DpknG-2: 5'-CCCATCCACTAAACTTAAACAATCTTCATTATCCTTCATCGTTTTC-3'; SEQ ID NO 6
DpknG-3: 5'-TGTTTAAGTTTAGTGGATGGGGCGAATGCCGTGCGGCCACT-3'; SEQ ID NO 7
DpknG-4: 5'-GATTCTAGAGTTAGCTATCGCTAGGTACG-3' ; SEQ ID NO 8

Die erfolgreiche Deletion des *pknG*-Gens wurde mittels PCR mit den Oligonukleotiden DpknG-5 (SEQ ID NO 9) und DpknG-6 (SEQ ID NO 10) kontrolliert:
DpknG-5: 5'-GCTGCGCGGTTTTGAAGTGG-3'; SEQ ID NO 9
DpknG-6: 5'-GATCGATCCAGAGCGTAACGC-3' SEQ ID NO 10

Es wurde das bei erfolgreicher Deletion erwartete 1364-bp-DNA-Fragment erhalten, nicht aber das im Wildtyp zu erwartende 3,76-kb-DNA-Fragment.

Außerdem wurde die erfolgreiche Deletion des *pknG-*Gens durch Southem-Blot-Analyse (Southern, E. M., J. Mol. Biol. 98: 503-517 (1975) überprüft, wobei das mit Digoxigenin-markierte "cross-over"-PCR-Produkt als Sonde verwendet wurde. In Hindlll-verdauter chromosomaler DNA des *C. glutamicum* Wildtyps ATCC13032 wurden wie erwartet hybridisierende DNA-Fragmente von 6,5 kb und 2,4 kb detektiert, in Hindlll-verdauter chromosomaler DNA der *pknG*-Mutante dagegen nur ein DNA-Fragment von 6,5 kb.

Für die Deletion des Gens *odhl* in *C. glutamicum* ATCC13032 wurden zur Amplifizierung der 5'- und 3'-flankierenden Bereiche des *odhl*-Gens die Oligonukleotid-Paare Dodhl-1 (SEQ ID NO 11) und Dodhl-2 (SEQ ID NO 12) (5'-Bereich) bzw. Dodhl-3 (SEQ ID NO 13) und Dodhl-4 (SEQ ID NO 14) (3'-Bereich) eingesetzt:
Dodhl-1 GACAAGCTTGATCAGTCCGTGAGGGAAC ; SEQ ID NO 11
Dodhl-2 CCCATCCACTAAACTTAAACAGCCGTTGTTGTCGCTCATTAAAC ; SEQ ID NO 12
Dodhl-3 TGTTTAAGTTTAGTGGATGGGAAGTTCCGCCTGGTTTTCCTCG ; SEQ ID NO 13
Dodhl-4 GTCGGATCCGAGGAGGAACCAAGGATG ; SEQ ID NO 14

Die erfolgreiche Deletion des *odhl-*Gens wurde durch Southern-Blot-Analyse (Southern, E. M., J. Mol. Biol. 98: 503-517 (1975) überprüft, wobei das mit Digoxigenin-markierte "cross-over"-PCR-Produkt als Sonde verwendet wurde. In Sphl-verdauter chromosomaler DNA des Wildtyps wurde wie erwartet ein hybridisierendes DNA-Fragment von 2,6 kb detektiert, in Sphl-verdauter chromosomaler DNA der *odhl-*Mutante dagegen nur ein DNA-Fragment von 2,3 kb. In Pstl-verdauter chromosomaler DNA des Wildtyps wurden wie erwartet hybridisierende Fragmente von 0,7 kb und 1,5 kb detektiert, in Pstl-verdauter chromosomaler DNA der *odhl-*Mutante dagegen nur ein Fragment von 1,8 kb.

### 2. Einfluss der Deletion von pknG in C. glutamicum ATCC13032 auf die internen Glutamat- und Glutamin-Konzentrationen.

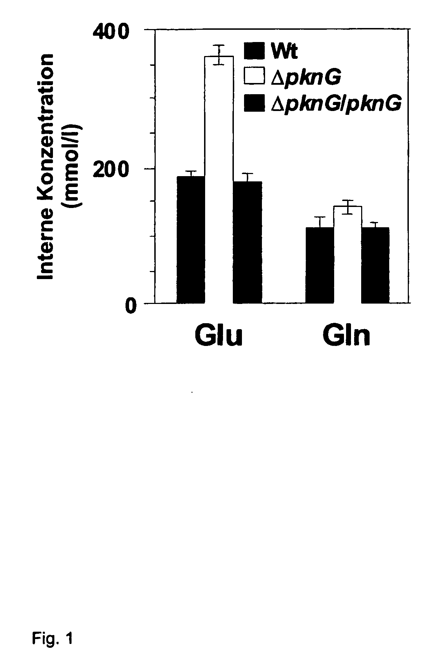

Die Stämme *C. glutamicum* ATCC13032 transformiert mit dem Plasmid pEKEx2 (Eikmanns et al, Gene 102: 93-98 (1991)), *C. glutamicum* Δ*pknG* transformiert mit pEKEx2 und *C. glutamicum* Δ*pknG* transformiert mit dem Plasmid pEKEx2-pknG (enthält das *C. glutamicum pknG*-Gen einschliesslich seiner eigenen Ribosomenbindungsstelle unter der Kontrolle des auf pEKEx2 vorhandenen Isopropylthiogalactosid (IPTG)-induzierbaren Promotors) wurden zunächst auf Him-Herz-Agar mit Kanamycin (25 mg/l) für 24 h bei 30 °C inkubiert. Ausgehend von dieser Agarplattenkultur wurden Vorkulturen angeimpft (5 ml Hirn-Herz-Medium mit 2% (w/v) Glucose, 25 mg/l Kanamycin und 1 mM IPTG in Reagenzgläsern). Diese Vorkulturen wurden 16 h bei 30°C und bei 170 Upm auf dem Schüttler inkubiert. Von diesen Vorkulturen wurden Hauptkulturen angeimpft, so daß die Anfangs-OD₆₀₀ von 1 erreicht wurde. Für die Hauptkulturen wurde ein modifiziertes CGXII-Minimalmedium (Keilhauer et al., J. Bacteriol. 175: 5595-5603 (1993) verwendet, das keinen Harnstoff und kein Ammoniumsulfat enthielt und als Kohlenstoff- bzw. Kohlenstoff- und Stickstoffquelle 5 mM Glucose und 100 mM L-Glutamin enthielt. Ausserdem wurden dem Medium 25 mg/l Kanamycin und 1 mM IPTG zugesetzt. Die Hauptkulturen wurden 12 Stunden bei 30°C und 150 rpm auf einem Schüttler inkubiert. Danach hatten die Kulturen eine optische Dichte bei 600 nm (OD₆₀₀) zwischen 4,0 bis 5,6 erreicht. Die Bestimmung der intrazellulären Konzentrationen von Glutamat und Glutamin erfolgte in Anlehnung an die von Wittmann et al. (Anal. Biochem. 327: 135-139 (2004)) beschriebene Filtrationsmethode, bei der die Zellen nicht gequencht werden. Dazu wurden Kulturvolumen mit 0,5 bis 1 mg Biotrockenmasse mittels Vakuum rasch durch Glasfaser-Filter (Millipore APFC02500) abfiltriert und die Filter-gebundenen Zellen viermal mit 0,9%-NaCl-Lösung (Raumtemperatur) gewaschen. Zur Extraktion der internen Metabolite wurden die Filter mit den daran gebundenen Zellen in 1,3 ml 50 µM Omithin-Lösung 15 Minuten bei 95 °C inkubiert. Nach Zentrifugation wurden die Aminosäuren im Überstand (Zellextrakt) wie oben beschrieben mittels HPLC quantifiziert. Für die Berechnung der zellinternen Konzentrationen wurde ein Zellvolumen von 1,5 ml/g Trockenzellen verwendet (Krämer et al., Eur. J. Biochem. 194: 929-935 (1990)). In Fig. 1 ist das Resultat dieses Versuches dargestellt. Die Standardabweichungen wurden aus drei unabhängigen Kultivierungen berechnet.

Aus Fig. 1 wird deutlich, dass der Stamm *C. glutamicum* Δ*pknG*/pEKEx2 (Δ*pknG* in Fig. 1) eine um 94% höhere interne Glutamat-Konzentration besitzt als der Vergleichsstamm *C. glutamicum*/pEKEx2 (Wt in Fig. 1). Durch Komplementation des Stammes *C. glutamicum* Δ*pknG* mit dem Plasmid pEKEx2-pknG (Δ*pknG*/*pknG* in Fig. 1) kann die Erhöhung der internen Glutamat-Konzentration wieder rückgängig gemacht werden. Im Fall von Glutamin ist die interne Konzentration im Stamm *C. glutamicum* Δ*pknG* um 27% gegenüber dem Parental-Stamm erhöht.

### 3. Einfluss der Deletion von pknG in C. g/utamicum ATCC13032 auf Wachstum und Glutamat-Bildung in Gegenwart von 500 mg/l Ethambutol als Induktor.

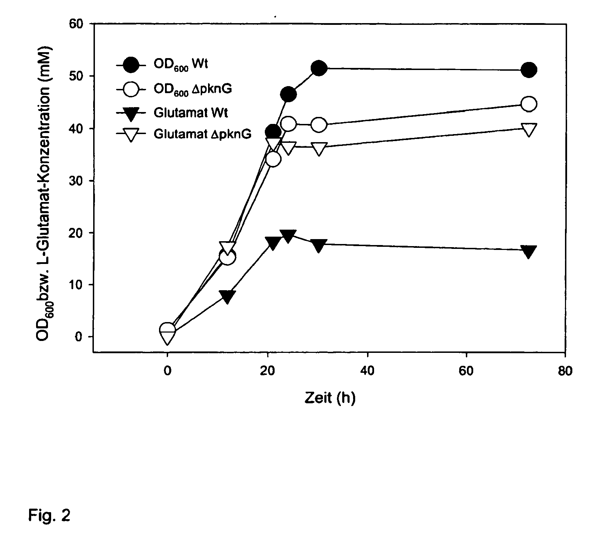

Die Stämme *C. glutamicum* ATCC13032 (Wt in Fig. 2) und *C. glutamicum* Δ*pknG* (DpknG in Fig. 2) wurden zunächst auf Him-Herz-Agar für 24 h bei 30°C inkubiert. Ausgehend von dieser Agarplattenkultur wurden Vorkulturen angeimpft (5 ml Him-Herz-Medium in Reagenzgläsern) und 6 h bei 30°C und 170 Upm auf einem Schüttler inkubiert. Von diesen Vorkulturen wurde eine zweite Serie von Vorkulturen mit 20 ml CGXII-Minimalmedium mit 5% (w/v) Glucose in 100-ml Erlenmeyer-Kolben angeimpft und 16 h bei 30 °C und 150 rpm auf einem Schüttler inkubiert. Ausgehend von diesen Vorkulturen wurden dann die Hauptkulturen in einem 500-ml Erlenmeyer-Kolben auf eine OD₆₀₀ von 1 angeimpft und für die angegebene Zeit bei 30°C und 150 rpm auf einem Schüttler inkubiert. Für die Hauptkultur wurde 50 ml CGXII-Minimalmedium (Keilhauer et al., J. Bacteriol. 175: 5595-5603 (1993) mit 5% Glucose (w/v) als Kohlenstoffquelle verwendet und das Medium mit 500 mg/l Ethambutol versetzt. Zu den angebenen Zeitpunkten wurden Proben für die Bestimmung der OD₆₀₀ sowie der Glutamat-Konzentration im Medium entnommen. Das Beispiel zeigt, dass Glutamat-Bildungsrate und Glutamat-Endkonzentration in der *pknG-*Deletionsmutante deutlich erhöht sind gegenüber dem Parental-Stamm. Die Zellausbeute ist dagegen in der Mutante erniedrigt.

### 4. Konstruktion von Plasmiden zur Expression von unmutiertem und mutiertem Odhl-Protein in C. glutamicum

Für die Expression des unmutierten Odhl-Proteins in *C. glutamicum* wurde das *odhl*-Gen von *C. glutamicum* mit den Oligonukleotiden odhl-for-2 und odhl-rev-2 ausgehend von chromosomaler DNA von *C. glutamicum* ATCC13032 mittels PCR amplifiziert. Dabei wurde über das Oligonukleotid odhl-rev-2 eine StrepTag-II-kodierende Sequenz (Skerra, A. und Schmidt, T.G.M., Methods Enzymol. 326: 271-304) vor dem Stopcodon des *odhl*-Gens eingeführt. Über die mit den Oligonukleotiden odhl-for-2 und odhl-rev-2 eingeführten BamHl-Schnittstellen wurde das PCR-Produkt in den *Escherichia coli-C. g*/*utamicum-Shuttlevektor* pJC1 (Cremer, J. et al., Mol. Gen. Genet. 220: 478-480 (1990)) kloniert, wobei *E*. *coli* DH5α (Invitrogen) als Wirtsbakterium diente.

| | |
|---|---|
| odhI-for-2: | 5'-GACGGATCCTGAGAACTATGAAATTCC-3'; SEQ ID NO 15 |
| odhI-rev-2: | |

Die Odhl-kodierende Sequenz des rekombinanten Plasmids (pJC1-odhl) wurde mittels DNA-Sequenzanalyse untersucht und auf diese Weise gezeigt, dass sie mit Ausnahme der StrepTag-II-kodierenden Sequenz identisch war mit der Odhl-kodierenden Sequenz im Genom von *C. glutamicum* ATCC13032 (Kalinowski, J. et al., J. Biotechnol. 104: 5-25). Das Plasmid pJC1-odhl wurde anschliessend über Elektroporation in die gewünschten *C. glutamicum*-Stämme transferiert und die entsprechenden Stämme in Medien mit 25 mg/l Kanamycin kultiviert.

Für die Expression eines mutierten Odhl-Proteins in *C. glutamicum,* bei dem das ACC-Codon für den Threonin-Rest an Position 14 des Odhl-Proteins von *C. glutamicum* gegen ein GCC-Codon, das für Alanin kodiert, ausgetauscht wurde, wurden zunächst PCR-Produkte hergestellt mit den Oligonukleotidpaaren odhI-for-2 und T14A-rev sowie T14A-for und odhI-rev-2 und chromosomaler DNA von *C. glutamicum ATCC13032 .* Anschliessend wurden die beiden erhaltenen PCR-Produkte über "cross-over"-PCR mit den Oligonukleotiden odhI-for-2 und odhI-rev-2 fusioniert und über die BamHl-Schnittstellen, die mit den beiden letztgenannten Oligonukleotiden eingeführt wurden, in den Vektor pJC1 kloniert. Dabei wurde wiederum *E. coli* DHSα als Wirtsorganismus verwendet.

| | |
|---|---|
| T14A-for | GCCACAGGTCGAGGCCACCTCAGTATTCC; SEQ ID NO 17 |
| T14A-rev | GGAATACTGAGGTGGCCTCGACCTGTGGC ; SEQ ID NO 18 |

Die Odhl-kodierende Sequenz des rekombinanten Plasmids (pJC1-odhl-T14A) wurde mittels DNA-Sequenzanalyse untersucht und auf diese Weise gezeigt, dass sie mit Ausnahme der gewünschten Mutation des Odhl-Codons 14 (GCC anstelle von ACC) und der StrepTag-II-kodierenden Sequenz identisch war mit der Odhl-kodierenden Sequenz im Genom von *C. glutamicum* ATCC13032 (Kalinowski, J. et al., J. Biotechnol. 104: 5-25). Das Plasmid pJC1-odhl-T14A wurde anschliessend über Elektroporation in die gewünschten *C. glutamicum-*Stämme transferiert und die entsprechenden Stämme in Medien mit 25 mg/l Kanamycin kultiviert.

### 5. Einfluss eines Odhl-Proteins mit einem Threonin-14 zu Alanin-Austausch auf Wachstum (OD₆₀₀) und Glutamat-Bildung

Ein Stamm von *Corynebacterium glutamicum* ATCC13032, in dem das chromosomale *odhI*-Gen durch die oben beschriebene Methode (Niebisch and Bott, Arch. Microbiol. 175: 282-294 (2001)) deletiert worden war, wurde mit dem Plasmid pJC1-odhl-T14A transformiert. Dieses Plasmid trägt das *C. glutamicum odhI*-Gen unter der Kontrolle seines nativen Promotors und seiner nativen Ribosomenbindungsstelle im Vektor pJC1 (Cremer et al., Mol. Gen. Genet. 220: 478-480 (1990)). Aufgrund der Mutation des Threonin-Restes an Position 14 zu Alanin kann das plasmidkodierte Odhl-T14A-Protein nicht mehr durch die Proteinkinase PknG phosphoryliert werden. Als Vergleichstamm wurde der Wildtyp-Stamm *C. glutamicum* ATCC13032 transformiert mit dem Plasmid pJC1 verwendet.

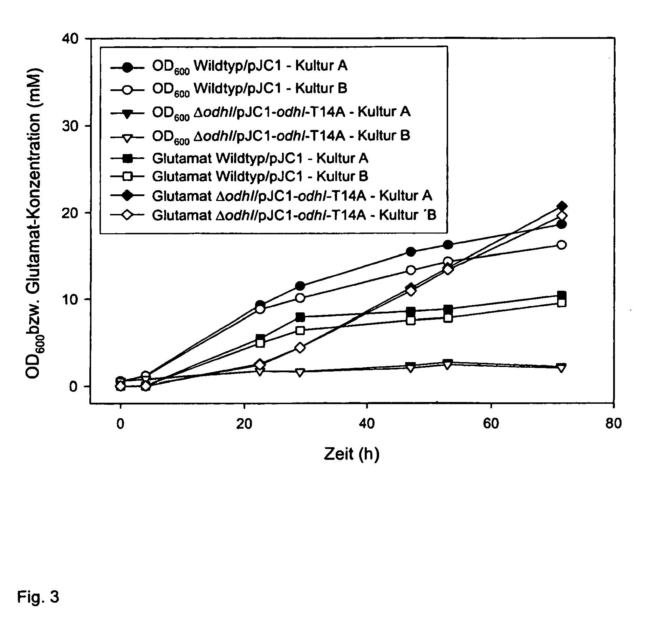

Die Stämme *C. glutamicum* ATCC13032/pJC1 (Wt in Fig. 3)) und *C. glutamicum* Δ*odhI*/pJC1-*odhI-*T14A wurden zunächst auf Him-Herz-Agar mit 25 mg/l Kanamycin für 24 h bei 30°C inkubiert. Ausgehend von dieser Agarplattenkultur wurden Vorkulturen angeimpft (20 ml LB-Medium mit 2% (w/v) Glucose und 25 mg/l Kanamycin in 100-ml Erlenmeyer-Kolben) und 16 h bei 30 °C und 150 Upm auf einem Schüttler inkubiert. Ausgehend von diesen Vorkulturen wurden dann die Hauptkulturen in einem 500-ml-Erlenmeyer-Kolben auf eine OD₆₀₀ von 1 angeimpft und für die angegebene Zeit bei 30°C und 150 rpm auf einem Schüttler inkubiert. Für die Hauptkultur wurde 50 ml CGXII-Minimalmedium (Keilhauer et al., J. Bacteriol. 175: 5595-5603 (1993) mit 5% Glucose (w/v) als Kohlenstoffquelle und 25 mg/l Kanamycin verwendet und das Medium mit 500 mg/l Ethambutol versetzt. Zu den angebenen Zeitpunkten wurden Proben für die Bestimmung der OD₆₀₀ sowie der Glutamat-Konzentration im Medium entnommen. Es wurden jeweils zwei unabhängige Kulturen der beiden Stämme analysiert.

Der Stamm *C. glutamicum* Δ*odhI*/pJC1-*odhI*-T14A wächst sehr schlecht, von einer anfänglichen OD₆₀₀ von 0.5 bis zu einer OD₆₀₀ von etwa 2. Er bildet aber in dieser Zeit etwa doppelt soviel Glutamat wie der Stamm *C. glutamicum*/pJC1, der in diesem Versuch bis zu einer OD₆₀₀ von 16-19 wächst. Daraus lässt sich berechnen, daß der Stamm *C. glutamicurn* pJC1 im Durchschnitt 2,3 mmol Glutamat pro g Trockenzellen bildet, der Stamm C. *glutamicum* Δ*odhI* /pJC1-*odhI*-T14A dagegen im Durchschnitt 37,63 mmol Glutamat pro g Trockenzellen. Eine OD₆₀₀ von 1 entspricht 0.25 g Trockenzellen pro Liter.

### 6. Phosphorylierung des Odhl-Proteins durch Proteinkinase G am Threonin-Rest 14

Um die Phosphorylierung des Odhl-Proteins durch die Proteinkinase G zu zeigen, wurde das Odhl-Protein modifiziert durch einen carboxyterminalen StrepTag-11 (Skerra, A. und Schmidt, T.G.M., Methods Enzymol. 326: 271-304) mit Hilfe des Plasmids pAN3K-*odhI* in *E. coli* DH5α überproduziert und anschliessend über Affinitätschromatographie an StrepTactin-Sepharose nach Angaben des Herstellers (IBA GmbH, Göttingen, Deutschland) bis zur apparenten Homogenität gereinigt. Auf die gleiche Weise wurden zwei mutierte Odhl-Proteine gereinigt, nämlich Odhl-T14A, bei dem der Threonin-Rest an Position 14 gegen Alanin ausgetauscht worden war, und Odhl-T15A, bei dem der Threonin-Rest an Position 15 gegen Alanin ausgetauscht worden war. Das PknG-Protein modifiziert durch einen carboxyterminalen StrepTag-II wurde mit Hilfe des Plasmids pEKEx2-*pknG*_{St} in *C. glutamicum* ATCC13032 überproduziert und anschliessend über Affinitätschromatographie an StrepTactin-Sepharose nach Angaben des Herstellers (IBA GmbH, Göttingen, Deutschland) bis zur apparenten Homogenität gereinigt.

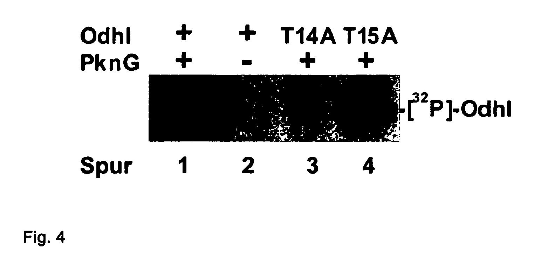

Die gereinigten Proteine Odhl, Odhl-T14A und Odhl-T15A (je 1 µg) wurden mit gereinigtem PknG-Protein (0,5 µg) und [γ-³²P]-ATP 30 min bei 37 °C inkubiert. Ausserdem wurde gereinigtes Odhl-Protein (1 µg) in Abwesenheit von gereinigtem PknG-Protein mit [γ-³²P]-ATP 30 min bei 37 °C inkubiert. Anschliessend wurden die Proteine über SDS-Polyacrylamid-Gelelektrophorese aufgetrennt, die Gele getrocknet und mit einem Phosphorimager analysiert. Das Ergebnis ist in Fig. 4 dargestellt. Es zeigt, dass die Proteine Odhl und Odhl-T15A durch PknG phosphoryliert werden, das Protein Odhl-T14A aber nicht. Ausserdem wird gezeigt, daß das Odhl-Protein keine Autophosphorylierungsaktivität besitzt.

### 7. Einfluss von phosphoryliertem und unphosphorvliertem Odhl-Protein auf die Aktivität des 2-Oxoglutarat-Dehydrogenase-Komplexes

Zum Nachweis der Inhibierung des 2-Oxoglutarat-Dehydrogenase-Komplexes durch das Odhl-Protein wurde ein zellfreier Extrakt aus Zellen des Stammes C. *glutamicum* Δ*pknG* Δ*odhI* hergestellt, bei dem die Gene für die Proteinkinase G und das Odhl-Protein deletiert sind. In diesen Zellextrakten sind also weder das PknG-Protein noch das Odhl-Protein enthalten. Der zellfreie Extrakt wurde über eine PD-10-Säule (Amersham Pharmacia) chromatographiert und auf diese Weise die Proteine von niedermolekularen Bestandteilen der Zellextraktes getrennt.

Die Aktivität der 2-Oxoglutarat-Dehydrogenase-Aktivität wurde bei 30°C in einem Testpuffer mit 50 mM TES 7,7, 3 mM L-Cystein, 10 mM MgCl₂, 0,9 mM Thiamindiphosphat, 0,2 mM Coenzym A, 2 mM NAD⁺ und 1,5 mM 2-Oxoglutarat spektrophotometrisch anhand der Zunahme der Absorption bei 340 nm gemessen. Für die Berechnung der spezifischen Aktivität wurde ein Extinktionskoeffizient für NADH bei 340 nm von 6,3 mM⁻¹ cm⁻¹ verwendet. Eine Aktivität von 1 U entspricht der Bildung von 1 µmol NADH pro Minute.

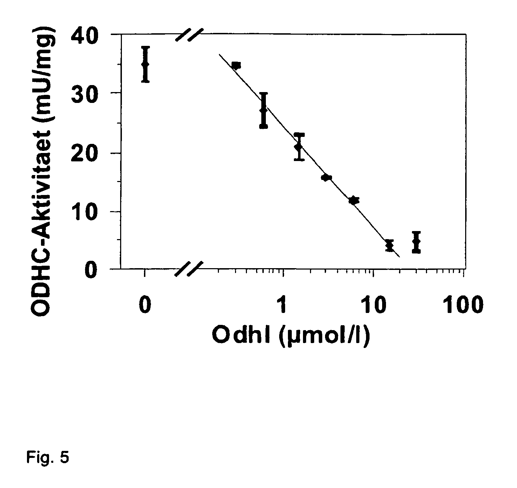

In Abwesenheit von Odhl besass der Zellextrakt eine 2-Oxoglutarat-Dehydrogenase-Komplex-Aktivität von ca. 35 mU/mg Protein (35 nmol min⁻¹ (mg Protein)⁻¹). Durch Zugabe von gereinigtem, unphosphoryliertem Odhl-Protein wurde die 2-Oxoglutarat-Dehydrogenase-Komplex-Aktivität gehemmt, und zwar in Abhängigkeit von der Odhl-Konzentration (Fig. 5).

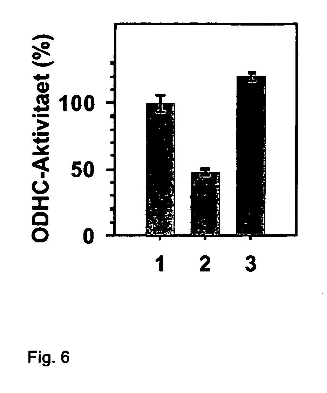

In einem weiteren Experiment zum Einfluss des Odhl-Proteins auf die Aktivität des 2-Oxoglutarat-Dehydrogenase-Komplexes wurde dieser Komplex aus einem *C. glutamicum*-Stamm angereichert (*C. glutamicum odhA*_{St}), in dem das OdhA-Protein für die E1-Untereinheit von ODHC einen carboxyterminalen StrepTag-II enthielt. Die Anreicherung des 2-Oxoglutarat-Dehydrogenase-Komplexes erfolgte über Affinitätschromatographie an StrepTactin-Sepharose nach den Angaben des Herstellers (IBA GmbH, Göttingen). Der angereicherte Komplex besass eine 2-Oxoglutarat-Dehydrogenase-Aktivität von 2,5 U/mg Protein. 2 µg des angereicherten ODHC-Komplexes wurden entweder ohne weitere Zusätze (Ansatz 1), oder mit 0,2 µg unphosphoryliertem Odhl-Protein und 2 mM ATP (Ansatz 2), oder mit 0,2 µg unphosphoryliertem Odhl-Protein, 2,2 µg PknG-Protein und 2 mM ATP (Ansatz 3) für 60 Minuten bei 30 °C inkubiert und anschliessend die 2-Oxoglutarat-Dehydrogenase-Aktivität bestimmt. Wie in Fig. 6 dargestellt, führte die Zugabe von unphosphoryliertem Odhl-Protein zu einer Hemmung der 2-Oxoglutarat-Dehydrogenase-Aktivität, während die Zugabe von Odhl, PknG und ATP nicht zu einer Hemmung führte.

### 8. Einfluss der Deletion des odhI-Gens auf die Glutamat-Produktion durch Corynebacterium glutamicum

Die Stämme *C. glutamicum* ATCC13032 (Wildtyp) und *C. glutamicum* Δo*dhI* wurden ausgehend von Glycerinkulturen auf Hirn-Herz-Agar-Platten ausgestrichen und über Nacht bei 30 °C inkubiert. Für jede Hauptkultur wurde eine Vorkultur angesetzt, für die 30 ml Him-Herz-Flüssigkmedium mit einer Einzelkolonie von Platte beimpft wurde. Die Vorkulturen wurden 15-18 h bei 30 °C und 150 Upm inkubiert, anschliessend die Zellen durch Zentrifugation geerntet, einmal mit 0.9% NaCl gewaschen und dann in 5 - 10 ml 0,9% NaCl resuspendiert. Die optische Dichte bei 600 nm (OD₆₀₀) dieser Suspensionen wurde bestimmt.

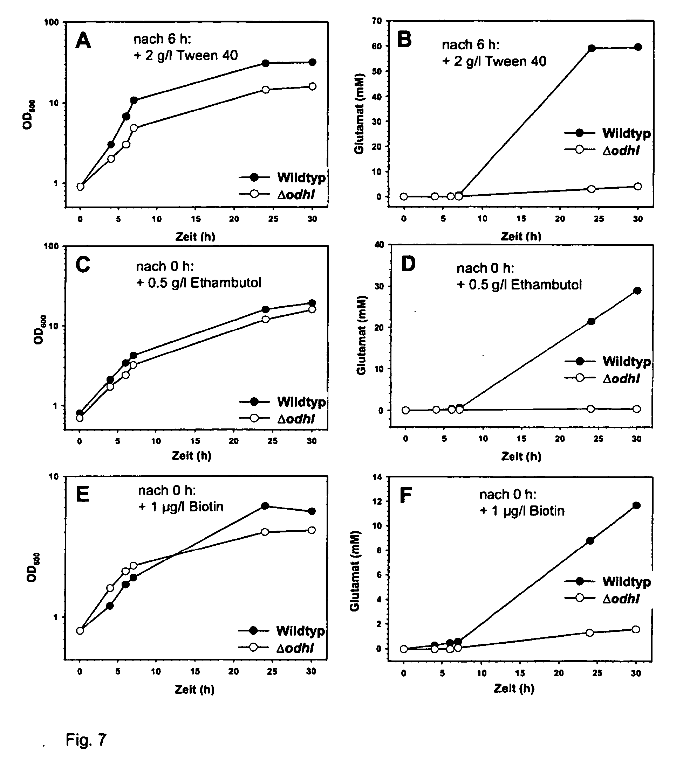

Wenn die Glutamat-Produktion durch Zugabe von Tween-40 (Fig. 7, A und B) oder Ethambutol (Fig. 7, C und D) ausgelöst wurde, wurden mit den genannten Suspensionen die Hauptkulturen auf eine OD₆₀₀ von 0,5 - 0,9 angeimpft. Die Hautptkultur enthielt 60 ml CGXII-Minimalmedium mit 4% (w/v) in 500-ml Erlenmeyerkolben mit zwei Schikanen und wurde bei 30 °C und 150 Upm inkubiert. Für das Auslösen der Glutamat-Bildung wurde den Kulturen entweder nach 6 bis 7 h 2 g/l Tween-40 zugegeben oder unmittelbar nach Inokulation 500 mg/l Ethambutol. Wenn die Glutamat-Produktion durch Biotin-Limitierung ausgelöst wurde (Fig. 7, E und F), wurden mit den genannten Suspensionen weitere Vorkulturen in CGXII-Minimalmedium mit 4% Glucose, aber nur 2,5 µg/mi Biotin inokuliert (reguläres CGXII-Minimalmedium enthält 300 µg/l Biotin). Diese Vorkulturen wurden exakt 24 h bei 30 °C und 150 Upm inkubiert, anschliessend die Zellen abzentrifugiert, gewaschen und in 0,9% NaCl resuspendiert. Mit diesen Suspensionen wurden dann die Hauptkulturen auf eine OD600 von 0,5 - 0,9 angeimpft. Für die Hauptkulturen wurde CGXII-Minimalmedium mit 4% Glucose und 1 µg/l Biotin verwendet. Zu den Zeitpunkten 0 h, 4 h, 6 h, 7 h, 24 h und 30 h wurden 1-ml-Proben der Hauptkultren entnommen und die OD₆₀₀ sowie die Glutamat-Konzentration im zellfreien Überstand bestimmt.

In Fig. 7 sind das Wachstum und die Glutamat-Bildung der oben beschriebenen Hauptkulturen dargestellt. Die Werte stellen Mittelwerte aus mindestens vier unabhängigen Kulturen dar. Es wird deutlich, dass der Stamm *C. glutamicum* Δ*odhI* sowohl in Gegenwart von Tween-40 (Fig. 7A und 7B) als auch in Gegenwart von Ethambutol (Fig. 7C und 7D) als auch bei Biotin-Limitierung (Fig. 7E und 7F) kein oder nur sehr geringe Konzentrationen an Glutamat im Vergleich zum Wildtyp bildet. Diese Ergebnisse bestätigen, dass das Odhl-Protein entscheidend für eine effiziente Glutamat-Bildung ist.

Beschreibung der Figuren:
Fig. 1. Zellinterne Konzentrationen von Glutamat (Glu) und Glutamin (Gln) in den Stämmen *C. glutamicum*/pEKEx2 (Wt), *C. glutamicum OpknG*/*pEKEx2* (Δ*pknG*) und *C. glutamicum* Δ*pknG*/pEKEx2-*pknG* (Δ*pknG*)/*pknG*). Die Standardabweichung aus drei unabhängigen Kultivierungen ist ebenfalls angegeben.
Fig. 2: Wachstum (OD₆₀₀) und Glutamat-Bildung durch *C. glutamicum* ATCC13032 (Wt) und *C. glutamicum* Δ*pknG*-Mutante (ΔpknG). Die Werte stellen Mittelwerte von drei unabhängigen Kultivierungen dar, die Standardabweichung betrug weniger als 20%.
Fig. 3. Wachstum (OD₆₀₀) und Glutamat-Bildung durch *C. glutamicum* ATCC13032/pJC1 (Wildtyp/pJC1) und *C. glutamicum* Δ*odhI*/pJC1-*odhI*-T14A (Δ*odhI*/pJC1-*odhI*-T14A). Es sind die Werte von je zwei unabhängigen Kultivierungen dargestellt.
Fig. 4. Nachweis der Phosphorylierung der Proteine Odhl und Odhl-T15A durch die Proteinkinase G. Die Protein Odhl und PknG (Spur 1), Odhl (Spur 2), Odhl-T14A und PknG (Spur 3) sowie OdhI-T15A und PknG (Spur 4) wurden mit [γ-³²P]-ATP 30 min bei 37 °C inkubiert und anschliessend über SDS-Polyacrylamidgelelektrophorese aufgetrennt. Das getrocknete SDS-Gel wurde mit einem Phosphorimager analysiert.
Fig. 5. Hemmung der Aktivität des 2-Oxoglutarat-Dehydrogenase-Komplexes im Zellextrakt des Stammes *C. glutamicum* Δ*pknG* Δ*odhI* durch gereinigtes, unphosphoryliertes Odhl-Protein.
Fig. 6. Aktivität des angereicherten 2-Oxoglutarat-Dehydrogenase-Komplexes in Abwesenheit von Zusätzen (Nr. 1), in Anwesenheit von unphosphoryliertem Odhl-Protein (Nr. 2), und in Anwesenheit von Odhl, PknG und ATP (Nr. 3). In Anwesenheit von PknG und ATP wird Odhl phosphoryliert und hemmt die Aktivität von ODHC nicht, während das unphosphorylierte Odhl-Protein hemmt.
Fig. 7. Vergleich des Wachstums (A, C, E) und der Glutamat-Bildung (B, D, F) durch die Stämme *C. glutamicum* ATCC13032 (Wildtyp) und *C. glutamicum* Δ*odhI* bei Zugabe von 2 g/l Tween-40 (A und B), bei Zugabe von 0.5 g/l Ethambutol (C und D) sowei bei Biotin-Limitierung (1 µg/l) (E und F). Die Stämme wurden in CGXII-Minimalmedium mit 4% (w/v) Glucose bei 30°C und 150 Upm kultiviert. Das Wachstum wurde anhand der optischen Dichte bei 600 nm bestimmt (OD₆₀₀), die Glutamat-Konzentration im Kulturüberstand wurde HPLCanalytisch bestimmt.

## Patentansprüche

1. Verfahren zur Produktion von Aminosäuren in aminosäure-produzierenden Mikroorganismen, bei dem die Aktivität des 2-Oxoglutarat-Dehydrogenase-Komplexes durch ein Protein vermindert wird, **dadurch gekennzeichnet, dass** das Protein nicht phosphoryliert ist und durch eine Nukleinsäuresequenz codiert wird umfassend
a) eine Nukleinsäuresequenz mit der in SEQ ID NO:1 dargestellten Nukleinsäuresequenz aus *C. glutamicum*; oder
b) eine Nukleinsäuresequenz, die sich aufgrund des degenerierten genetischen Codes durch Rückübersetzung aus der in SEQ ID NO:2 aus *C. glutamicum* dargestellten Aminosäuresequenz ableiten lässt; oder
c) eine Nukleinsäuresequenz, welche eine Identität mit der SEQ ID NO:1 aus *C. glutamicum* von mindestens 50% aufweist, und die Expression eines Proteins mit den gleichen Eigenschaften wie die des Proteins aus *C. glutamicum* bewirkt, wobei das Protein die Aktivität des 2-Oxoglutarat-Dehydrogenase-Komplexes inhibiert, aber nicht die Expression der für den 2-Oxoglutarat-Dehydrogenase-Komplex kodierenden Gene beeinflußt.

2. Verfahren nach Anspruch 1 **dadurch gekennzeichnet, daß** die Mikroorganismen Stäbchenbakterien, bevorzugt gram-positive Stäbchenbakterien, besonders bevorzugt der Familie *Corynebacteriaceae* oder *Mycobacteriaceae* oder *Streptomycetaceae* sind.

3. Verfahren nach Anspruch 1 **dadurch gekennzeichnet, daß** die Mikroorganismen aus der Gattung *Corynebacterium* stammen.

4. Verfahren nach Anspruch 1 **dadurch gekennzeichnet, daß** die Mikroorganismen *Corynebacterium* glutamicum oder *Corynebacterium efficiens* sind.

5. Verfahren nach Anspruch 1 **dadurch gekennzeichnet, daß** die Produktion von Glutamat, Glutamin, Aspartat, Ornithin, Asparagin, Arginin, Lysin, Valin, Leucin, Isoleucin, Methionin, Cystein und/oder Prolin, bevorzugt Glutamat und/oder Glutamin, besonders bevorzugt Glutamat erhöht wird.

6. Verfahren nach Anspruch 1 **dadurch gekennzeichnet, daß** die Produktion des L-Isomers der Aminosäure erhöht wird.

7. Verfahren nach Anspruch 1 **dadurch gekennzeichnet, daß** das Protein an den Aminosäuren Threonin an den Positionen 14 und/oder 15, bevorzugt 14 nicht phosphoryliert ist.

8. Verfahren nach Anspruch 1 **dadurch gekennzeichnet, daß** die Aktivität der Proteinkinase PknG vermindert wird.

9. Verfahren nach Anspruch 1 **dadurch gekennzeichnet, daß** die Aktivität der Proteinkinase PknG durch Deletion der Nukleinsäuresequenz umfassend:
a) eine Nukleinsäuresequenz mit der in SEQ ID NO:3 dargestellten Nukleinsäuresequenz; oder
b) eine Nukleinsäuresequenz, die sich aufgrund des degenerierten genetischen Codes durch Rückübersetzung aus der in SEQ ID NO:4 dargestellten Aminosäuresequenz ableiten lässt; oder
c) eine Nukleinsäuresequenz, welche eine Identität mit der SEQ ID NO:3 von mindestens 50% aufweist, vermindert wird.

10. Verfahren nach Anspruch 1 **dadurch gekennzeichnet, daß** die Mikroorganismen unter Bedingungen kultiviert werden, die eine Induktion der Glutamat-Überproduktion bewirken, durch Biotinmangel, Detergenzien- oder Penicillin-Zugabe, Ethambutol-Zugabe, Ausschalten des dtsR1-Gens oder anderer Gene der Fettsäurebiosynthese, oder Temperatur-erhöhung.

11. Verwendung eines Proteins zur Kontrolle der Aktivität des 2-Oxogluturat-Dehydrogenase-Komplexes in Mikroorganismen, wobei das Protein codiert ist durch eine Nukleinsäuresequenz umfassend:
a) eine Nukleinsäuresequenz mit der in SEQ ID NO:1 dargestellte Nukleinsäuresequenz aus *C. glutamicum*; oder
b) eine Nukleinsäuresequenz, die sich aufgrund des degenerierten genetischen Codes durch Rückübersetzung aus der in SEQ ID NO:2 aus *C. glutamicum* dargestellten Aminosäuresequenz ableiten lässt; oder
c) eine Nukleinsäuresequenz, welche eine Identität mit der SEQ ID NO:1 aus *C. glutamicum* von mindestens 50% aufweist und die Expression eines Proteins mit den gleichen Eigenschaften wie die des Proteins aus *C. glutamicum* bewirkt, wobei das Protein die Aktivität des 2-Oxoglutarat-Dehydrogenase-Komplexes inhibiert, aber nicht die Expression der für den 2-Oxoglutarat-Dehydrogenase-Komplex kodierenden Gene beeinflußt.

## Claims

1. Method for producing amino acids in amino acid-producing microorganisms, which comprises the activity of the 2-oxoglutarate dehydrogenase complex being reduced by a protein, **characterized in that** the protein is in a non-phosphorylated state and is encoded by a nucleic acid sequence comprising
a) a nucleic acid sequence having the *C. glutamicum* nucleic acid sequence depicted in SEQ ID NO: 1; or
b) a nucleic acid sequence which can be derived by way of back translation from the amino acid sequence depicted in SEQ ID NO: 2 of *C. glutamicum,* owing to the degeneracy of the genetic code; or
c) a nucleic acid sequence which is at least 50% identical to SEQ ID NO: 1 of *C. glutamicum* and which effects expression of a protein having the same properties as those of the *C. glutamicum* protein, wherein said protein inhibits the activity of the 2-oxoglutarate dehydrogenase complex but does not influence expression of the genes coding for the 2-oxoglutarate dehydrogenase complex.

2. Method according to Claim 1, **characterized in that** the microorganisms are rod-shaped bacteria, preferably Gram-positive rod-shaped bacteria, particularly preferably of the *Corynebacteriaceae* or *Mycobacteriaceae* or *Streptomycetaceae* family.

3. Method according to Claim 1, **characterized in that** the microorganisms are from the genus *Corynebacterium.*

4. Method according to Claim 1, **characterized in that** the microorganisms are *Corynebacterium glutamicum* or *Corynebacterium efficiens.*

5. Method according to Claim 1, **characterized in that** production of glutamate, glutamine, aspartate, ornithine, asparagine, arginine, lysine, valine, leucine, isoleucine, methionine, cysteine and/or proline, preferably glutamate and/or glutamine, particularly preferably glutamate, is increased.

6. Method according to Claim 1, **characterized in that** production of the L-isomer of the amino acid is increased.

7. Method according to Claim 1, **characterized in that** the protein is not phosphorylated on the threonine amino acids in positions 14 and/or 15, preferably 14.

8. Method according to Claim 1, **characterized in that** the activity of the PknG protein kinase is reduced.

9. Method according to Claim 1, **characterized in that** the activity of the PknG protein kinase is reduced by deleting the nucleic acid sequence comprising:
a) a nucleic acid sequence having the nucleic acid sequence depicted in SEQ ID NO: 3; or
b) a nucleic acid sequence which can be derived by way of back translation from the amino acid sequence depicted in SEQ ID NO: 4, owing to the degeneracy of the genetic code; or
c) a nucleic acid sequence which is at least 50% identical to SEQ ID NO: 3.

10. Method according to Claim 1, **characterized in that** the microorganisms are cultured under conditions which induce glutamate overproduction due to biotin deficiency, addition of detergents or penicillin, addition of ethambutol, elimination of the dtsR1 gene or other genes of fatty acid biosynthesis, or temperature increase.

11. Use of a protein for controlling the activity of the 2-oxoglutarate dehydrogenase complex in microorganisms, wherein the protein is encoded by a nucleic acid sequence comprising:
a) a nucleic acid sequence having the nucleic acid sequence of C. glutamicum depicted in SEQ ID NO: 1; or
b) a nucleic acid sequence which can be derived by way of back translation from the amino acid sequence depicted in SEQ ID NO: 2 of C. glutamicum, owing to the degeneracy of the genetic code; or
c) a nucleic acid sequence which is at least 50% identical to SEQ ID NO: 1 of C. glutamicum and which effects expression of a protein having the same properties as those of the *C. glutamicum* protein, wherein said protein inhibits the activity of the 2-oxoglutarate dehydrogenase complex but does not influence expression of the genes coding for the 2-oxoglutarate dehydrogenase complex.

## Revendications

1. Procédé pour la production d'acides aminés dans des micro-organismes producteurs d'acides aminés, dans lequel l'activité du complexe 2-oxoglutarate déshydrogénase est diminuée par une protéine, **caractérisé en ce que** la protéine est non phosphorylée et est codée par une séquence d'acide nucléique comprenant
a) une séquence d'acide nucléique ayant la séquence d'acide nucléique de *C. glutamicum* présentée dans SEQ ID n° 1 ; ou
b) une séquence d'acide nucléique qui, en raison du code génétique dégénéré peut dériver par rétro-traduction de la séquence d'aminoacides présentée dans SEQ ID n° 2 de *C. glutamicum ;* ou
c) une séquence d'acide nucléique qui présente une identité d'au moins 50 % avec la séquence SEQ ID n° 1 de *C. glutamicum,* et qui induit l'expression d'une protéine ayant les mêmes propriétés que celles de la protéine de *C. glutamicum,* la protéine inhibant l'activité du complexe 2-oxoglutarate déshydrogénase, mais n'affectant pas l'expression des gènes codant pour le complexe 2-oxoglutarate déshydrogénase.

2. Procédé selon la revendication 1, **caractérisé en ce que** les micro-organismes sont des bacilles, de préférence des bacilles à Gram positif ; de façon particulièrement préférée de la famille des *Corynebacteriaceae* ou des *Mycobacteriaceae* ou des *Streptomycetaceae.*

3. Procédé selon la revendication 1, **caractérisé en ce que** les micro-organismes proviennent du genre *Corynebacterium.*

4. Procédé selon la revendication 1, **caractérisé en ce que** les micro-organismes sont *Corynebacterium glutamicum* ou *Corynebacterium efficiens.*

5. Procédé selon la revendication 1, **caractérisé en ce que** la production de glutamate, de glutamine, d'aspartate, d'ornithine, d'asparagine, d'arginine, de lysine, de valine, de leucine, d'isoleucine, de méthionine, de cystéine et/ou de proline, de préférence de glutamate et/ou de glutamine, de façon particulièrement préférée de glutamate, est augmentée.

6. Procédé selon la revendication 1, **caractérisé en ce que** la production de l'isomère L de l'acide aminé est augmentée.

7. Procédé selon la revendication 1, **caractérisé en ce que** la protéine est non phosphorylée sur les aminoacides thréonine à la position 14 et/ou à la position 15, de préférence à la position 14.

8. Procédé selon la revendication 1, **caractérisé en ce que** l'activité de la protéine-kinase PknG est diminuée.

9. Procédé selon la revendication 1, **caractérisé en ce que** l'activité de la protéine-kinase PknG est diminuée par délétion de la séquence d'acide nucléique comprenant :
a) une séquence d'acide nucléique ayant la séquence d'acide nucléique présentée dans SEQ ID n° 3 ; ou
b) une séquence d'acide nucléique qui, en raison du code génétique dégénéré peut dériver par rétro-traduction de la séquence d'aminoacides présentée dans SEQ ID n° 4 ; ou
c) une séquence d'acide nucléique qui présente une identité d'au moins 50 % avec la séquence SEQ ID n° 3.

10. Procédé selon la revendication 1, **caractérisé en ce qu'**on cultive les micro-organismes dans des conditions qui provoquent une induction de la surproduction de glutamate, par carence en biotine, addition de détergents ou de pénicilline, addition d'éthambutol, extinction du gène dtsR1 ou d'autres gènes de la biosynthèse des acides gras, ou élévation de la température.

11. Utilisation d'une protéine pour la régulation de l'activité du complexe 2-oxoglutarate déshydrogénase dans des micro-organismes, la protéine étant codée par une séquence d'acide nucléique comprenant :
a) une séquence d'acide nucléique ayant la séquence d'acide nucléique de *C. glutamicum* présentée dans SEQ ID n° 1 ; ou
b) une séquence d'acide nucléique qui, en raison du code génétique dégénéré peut dériver par rétro-traduction de la séquence d'aminoacides présentée dans SEQ ID n° 2 de *C. glutamicum ;* ou
c) une séquence d'acide nucléique qui présente une identité d'au moins 50 % avec la séquence SEQ ID n° 1 de *C. glutamicum,* et qui induit l'expression d'une protéine ayant les mêmes propriétés que celles de la protéine de *C. glutamicum,* la protéine inhibant l'activité du complexe 2-oxoglutarate déshydrogénase, mais n'affectant pas l'expression des gènes codant pour le complexe 2-oxoglutarate déshydrogénase.


### SEQUENCE LISTING

<110> Forschungszentrum Jülich
<120> Verfahren zur Produktion von Aminosäuren mit Mikroorganismen
<130> FZJ0501PCT
<160> 18
<170> PatentIn version 3.3
<210> 1
   <211> 432
   <212> DNA
   <213> Corynebacterium glutamicum
<220>
   <221> CDS
   <222> (1)..(432)
<400> 1
   atg agc gac aac aac ggc acc ccg gag cca cag gtc gag acc acc tca
   48
   Met Ser Asp Asn Asn Gly Thr Pro Glu Pro Gln Val Glu Thr Thr Ser
   1 5 10 15
   gta ttc cgc gct gat cta ctg aag gaa atg gag tcg agc acc ggt act
   96
   Val Phe Arg Ala Asp Leu Leu Lys Glu Met Glu Ser Ser Thr Gly Thr
   20 25 30
   gct cca gcc tcc aca gga gct gaa aac ctt cct gca gga tct gct ctt
   144
   Ala Pro Ala Ser Thr Gly Ala Glu Asn Leu Pro Ala Gly Ser Ala Leu
   35 40 45
   ctt gta gtc aag cgt gga cca aat gca ggc gct cga ttc ctt ctg gac
   192
   Leu Val Val Lys Arg Gly Pro Asn Ala Gly Ala Arg Phe Leu Leu Asp
   50 55 60
   cag cca acc acg acc gct ggt cgt cac cca gaa agt gac atc ttc ctt
   240
   Gln Pro Thr Thr Thr Ala Gly Arg His Pro Glu Ser Asp Ile Phe Leu
   65                  70                  75                  80
   gat gat gtc acc gtt tca cgt cgc cac gca gag ttc cgc atc aat gaa
   288
   Asp Asp Val Thr Val Ser Arg Arg His Ala Glu Phe Arg Ile Asn Glu
                   85                  90                  95
   ggt gaa ttt gaa gtc gtg gac gta ggg tcc ctc aac gga acc tac gtt
   336
   Gly Glu Phe Glu Val Val Asp Val Gly Ser Leu Asn Gly Thr Tyr Val
               100                 105                 110
   aac cgc gag cca cgc aac gct cag gtc atg cag acc ggt gat gag atc
   384
   Asn Arg Glu Pro Arg Asn Ala Gln Val Met Gln Thr Gly Asp Glu Ile
           115                 120                 125
   cag att ggc aag ttc cgc ctg gtt ttc ctc gca ggc cct gct gag taa
   432
   Gln Ile Gly Lys Phe Arg Leu Val Phe Leu Ala Gly Pro Ala Glu
       130                 135                 140
<210> 2
   <211> 143
   <212> PRT
   <213> Corynebacterium glutamicum
<400> 2
   Met Ser Asp Asn Asn Gly Thr Pro Glu Pro Gln Val Glu Thr Thr Ser
   1               5                   10                  15
   Val Phe Arg Ala Asp Leu Leu Lys Glu Met Glu Ser Ser Thr Gly Thr
               20                  25                  30
   Ala Pro Ala Ser Thr Gly Ala Glu Asn Leu Pro Ala Gly Ser Ala Leu
           35                  40                  45
   Leu Val Val Lys Arg Gly Pro Asn Ala Gly Ala Arg Phe Leu Leu Asp
       50                  55                  60
   Gln Pro Thr Thr Thr Ala Gly Arg His Pro Glu Ser Asp Ile Phe Leu
   65                  70                  75                  80
   Asp Asp Val Thr Val Ser Arg Arg His Ala Glu Phe Arg Ile Asn Glu
                   85                  90                  95
   Gly Glu Phe Glu Val Val Asp Val Gly Ser Leu Asn Gly Thr Tyr Val
               100                 105                 110
   Asn Arg Glu Pro Arg Asn Ala Gln Val Met Gln Thr Gly Asp Glu Ile
           115                 120                 125
   Gln Ile Gly Lys Phe Arg Leu Val Phe Leu Ala Gly Pro Ala Glu
       130                 135                 140
<210> 3
   <211> 2469
   <212> DNA
   <213> Corynebacterium glutamicum
<220>
   <221> CDS
   <222> (1)..(2469)
<400> 3
   atg aag gat aat gaa gat ttc gat cca gat tca cca gca acc gaa gct
   48
   Met Lys Asp Asn Glu Asp Phe Asp Pro Asp Ser Pro Ala Thr Glu Ala
   1 5 10 15
   gtt gcc ttc aac cct ttc gac gat gac gat gag gat gat tcc ccc gct
   96
   Val Ala Phe Asn Pro Phe Asp Asp Asp Asp Glu Asp Asp Ser Pro Ala
   20 25 30
   acc tca gcc gtt gcc ttt aac cct ttt gaa gat gac gat gac gac gat
   144
   Thr Ser Ala Val Ala Phe Asn Pro Phe Glu Asp Asp Asp Asp Asp Asp
   35 40 45
   gag ttc caa ggc gaa ggc cta gaa ttc ctg ctg cgc gac ctc gac aat
   192
   Glu Phe Gln Gly Glu Gly Leu Glu Phe Leu Leu Arg Asp Leu Asp Asn
   50 55 60
   ctg cga gcc acc caa ggt caa atg gtg gtg gaa caa cca gca gtt gaa
   240
   Leu Arg Ala Thr Gln Gly Gln Met Val Val Glu Gln Pro Ala Val Glu
   65 70 75 80
   gac agc ctc ggg tca gca tct gcg cat acg gag aca act gcg gcc tca
   288
   Asp Ser Leu Gly Ser Ala Ser Ala His Thr Glu Thr Thr Ala Ala Ser
   85 90 95
   ctg cgt ccc cgc cca gag gtg gat cca agt gag agg agt cgt cga caa
   336
   Leu Arg Pro Arg Pro Glu Val Asp Pro Ser Glu Arg Ser Arg Arg Gln
   100 105 110
   gca att tcg ctg ttc cgc gaa cgg cgc cgc gta agg cgc caa tcc cgc
   384
   Ala Ile Ser Leu Phe Arg Glu Arg Arg Arg Val Arg Arg Gln Ser Arg
   115 120 125
   cca gtt gct gat ggc atg gtg gaa ttg ccg ttc atc acc ccc aaa ccg
   432
   Pro Val Ala Asp Gly Met Val Glu Leu Pro Phe Ile Thr Pro Lys Pro
   130 135 140
   gaa gat gag ctg ctc atc gac ccg gaa aag aag cgc aaa cct ggt gtg
   480
   Glu Asp Glu Leu Leu Ile Asp Pro Glu Lys Lys Arg Lys Pro Gly Val
   145 150 155 160
   gca gcg ccg caa ctt gtc gcg ggc gat atc gtc gca gag caa tat gaa
   528
   Ala Ala Pro Gln Leu Val Ala Gly Asp Ile Val Ala Glu Gln Tyr Glu
   165 170 175
   gtc ctc ggc gtc atc gcg cac ggc ggc atg ggt tgg att tac ctc gcc
   576
   Val Leu Gly Val Ile Ala His Gly Gly Met Gly Trp Ile Tyr Leu Ala
   180 185 190
   aac gac cgc aat gtg tcc ggc cgc atc gtg gtg ctc aaa ggc atg atg
   624
   Asn Asp Arg Asn Val Ser Gly Arg Ile Val Val Leu Lys Gly Met Met
   195 200 205
   gcg caa tct tcc gtt caa gac caa ggc acc gct gaa gcc gaa cgc gaa
   672
   Ala Gln Ser Ser Val Gln Asp Gln Gly Thr Ala Glu Ala Glu Arg Glu
   210 215 220
   ttc ctc gcc gac atc acc cac ccc ggc atc gtg aag gcc tac aac ttc
   720
   Phe Leu Ala Asp Ile Thr His Pro Gly Ile Val Lys Ala Tyr Asn Phe
   225 230 235 240
   atc gac gac ccc cgc gtc ccc ggc gga ttc atc gtc atg gaa tac gtc
   768
   Ile Asp Asp Pro Arg Val Pro Gly Gly Phe Ile Val Met Glu Tyr Val
   245 250 255
   aac ggc ccc tcc ctg aaa gac cgc tgc aaa gcc caa ccc gac ggc gtg
   816
   Asn Gly Pro Ser Leu Lys Asp Arg Cys Lys Ala Gln Pro Asp Gly Val
   260 265 270
   ctc cgc gtc gac ctc gcc atc ggc tac atc ctc gaa ctc ctc ccc gcc
   864
   Leu Arg Val Asp Leu Ala Ile Gly Tyr Ile Leu Glu Leu Leu Pro Ala
   275 280 285
   atg gac tac ctg cac caa cgc ggc gta gtg tac aac gac ctc aaa ccc
   912
   Met Asp Tyr Leu His Gln Arg Gly Val Val Tyr Asn Asp Leu Lys Pro
   290 295 300
   gaa aac gtc atc gcc acc gaa gac caa gtt aaa ctc atc gac ctc ggc
   960
   Glu Asn Val Ile Ala Thr Glu Asp Gln Val Lys Leu Ile Asp Leu Gly
   305 310 315 320
   gcg gtt acc ggc atc ggc gca ttc ggc tac att tac ggc acc aaa gga
   1008
   Ala Val Thr Gly Ile Gly Ala Phe Gly Tyr Ile Tyr Gly Thr Lys Gly
   325 330 335
   ttc caa gca ccc gaa gta gcc acc cat ggc ccc tca atc tcc tcc gat
   1056
   Phe Gln Ala Pro Glu Val Ala Thr His Gly Pro Ser Ile Ser Ser Asp
   340 345 350
   att ttc acc atc gga cgc acc ctc gca gca ctc acc atg ccc ctc ccc
   1104
   Ile Phe Thr Ile Gly Arg Thr Leu Ala Ala Leu Thr Met Pro Leu Pro
   355 360 365
   gtt gaa gac ggt gtc ctc gca ccg ggc atc ccc tcg ccc aaa aat tca
   1152
   Val Glu Asp Gly Val Leu Ala Pro Gly Ile Pro Ser Pro Lys Asn Ser
   370 375 380
   cct ctt ctg cgc agg cat ttg tcg ttc tac cgc ctc ctg caa cgc gcc
   1200
   Pro Leu Leu Arg Arg His Leu Ser Phe Tyr Arg Leu Leu Gln Arg Ala
   385 390 395 400
   acc gcc gac gac ccc caa cac cga ttc cgc aac gtc agc gaa cta cgc
   1248
   Thr Ala Asp Asp Pro Gln His Arg Phe Arg Asn Val Ser Glu Leu Arg
   405 410 415
   acc caa ctc tac ggc gta ctg cgt gaa att ttg gca gtc cgc gac ggc
   1296
   Thr Gln Leu Tyr Gly Val Leu Arg Glu Ile Leu Ala Val Arg Asp Gly
   420 425 430
   aaa caa tac ccg cca cag cac tca cta ttc tcc cca cag cga agc acc
   1344
   Lys Gln Tyr Pro Pro Gln His Ser Leu Phe Ser Pro Gln Arg Ser Thr
   435 440 445
   ttt ggc acc aaa cac ctc gtg ttc cgc acc gac cgc atc atc gac ggc
   1392
   Phe Gly Thr Lys His Leu Val Phe Arg Thr Asp Arg Ile Ile Asp Gly
   450 455 460
   atc gaa cga caa gca cgc atc aca gca cca gaa att gtc tcc gcg ctg
   1440
   Ile Glu Arg Gln Ala Arg Ile Thr Ala Pro Glu Ile Val Ser Ala Leu
   465 470 475 480
   cct gtc cca ctc atc gac cgc acc gac ccc ggc gcc cgt atg ctc tcc
   1488
   Pro Val Pro Leu Ile Asp Arg Thr Asp Pro Gly Ala Arg Met Leu Ser
   485 490 495
   gga tcc tcc tat gca gaa ccc tcc gaa acc ctg gaa act ctg cgc aac
   1536
   Gly Ser Ser Tyr Ala Glu Pro Ser Glu Thr Leu Glu Thr Leu Arg Asn
   500 505 510
   tcc atg gaa gac gag caa tac cgc caa tca atc gag atc ccc ctc ggt
   1584
   Ser Met Glu Asp Glu Gln Tyr Arg Gln Ser Ile Glu Ile Pro Leu Gly
   515 520 525
   gtc gtc cga gcc ctc ctt gac cta ggc ttt acc acc gaa gca cgc caa
   1632
   Val Val Arg Ala Leu Leu Asp Leu Gly Phe Thr Thr Glu Ala Arg Gln
   530 535 540
   tgg ctc gaa acc cta gag gga cgc atc ggc gac gac tgg cga cac aaa
   1680
   Trp Leu Glu Thr Leu Glu Gly Arg Ile Gly Asp Asp Trp Arg His Lys
   545 550 555 560
   tgg ttc tcc gga atc acc tac ctc ctc ctc gac gac tac gcc acc gcc
   1728
   Trp Phe Ser Gly Ile Thr Tyr Leu Leu Leu Asp Asp Tyr Ala Thr Ala
   565 570 575
   caa gta ttc ttc aac cac gtc ctg acc atc ctg ccc ggc gaa gcc gct
   1776
   Gln Val Phe Phe Asn His Val Leu Thr Ile Leu Pro Gly Glu Ala Ala
   580 585 590
   cct aaa cta gcc ctc gca gct gtt gac gaa ctc atc ctc caa caa atc
   1824
   Pro Lys Leu Ala Leu Ala Ala Val Asp Glu Leu Ile Leu Gln Gln Ile
   595 600 605
   ggc gcc gaa tcc acc gcc tat ctc acc cca gac atc gtc tct gca acc
   1872
   Gly Ala Glu Ser Thr Ala Tyr Leu Thr Pro Asp Ile Val Ser Ala Thr
   610 615 620
   gcg acc ctc agc aaa gat ttc gaa gac ctc gac gcc tcc gcc ttc gaa
   1920
   Ala Thr Leu Ser Lys Asp Phe Glu Asp Leu Asp Ala Ser Ala Phe Glu
   625 630 635 640
   tca ctc agc gac acc tgg tcc cac atc tcc agc gac cca cac gta gtc
   1968
   Ser Leu Ser Asp Thr Trp Ser His Ile Ser Ser Asp Pro His Val Val
   645 650 655
   cgc ttc cat tca ctg cgc ctc tac gca ctt gtc tgg gca acc aac ccc
   2016
   Arg Phe His Ser Leu Arg Leu Tyr Ala Leu Val Trp Ala Thr Asn Pro
   660 665 670
   acc acc gtg tcc tcc gcg ttc ggg ctc gcc cgc caa ctc atg gcc gaa
   2064
   Thr Thr Val Ser Ser Ala Phe Gly Leu Ala Arg Gln Leu Met Ala Glu
   675 680 685
   aac caa atc gaa ctc gca gtc caa gcc cta gac aaa ctc ccc caa tca
   2112
   Asn Gln Ile Glu Leu Ala Val Gln Ala Leu Asp Lys Leu Pro Gln Ser
   690 695 700
   tcc acc cac tac cga atg gcc acc ctc acc acc atc ttg ttg ctg gtc
   2160
   Ser Thr His Tyr Arg Met Ala Thr Leu Thr Thr Ile Leu Leu Leu Val
   705 710 715 720
   agc tcc aat ttg agt gaa tcc cgc atc cga cgg gct gcc cgc cga ctc
   2208
   Ser Ser Asn Leu Ser Glu Ser Arg Ile Arg Arg Ala Ala Arg Arg Leu
   725 730 735
   acc gaa atc ccc aca aac gaa ccc cgc ttc aac caa atc aaa att gcc
   2256
   Thr Glu Ile Pro Thr Asn Glu Pro Arg Phe Asn Gln Ile Lys Ile Ala
   740 745 750
   atc atg tcg gca ggc ctc agc tgg ctt cga gag cga aaa ctc aaa gct
   2304
   Ile Met Ser Ala Gly Leu Ser Trp Leu Arg Glu Arg Lys Leu Lys Ala
   755 760 765
   tcc gcc tcc gcg aac cct ttg ttt gaa tac ccg ttc tcc caa aaa ggc
   2352
   Ser Ala Ser Ala Asn Pro Leu Phe Glu Tyr Pro Phe Ser Gln Lys Gly
   770 775 780
   ctg cgc acc ggc atc tcc gag gca ctc cgc att cag gca cgt tct gca
   2400
   Leu Arg Thr Gly Ile Ser Glu Ala Leu Arg Ile Gln Ala Arg Ser Ala
   785 790 795 800
   ccg ttc ccg cac cac cgt tac gca ctt gtg gat atg gcg aat gcc gtg
   2448
   Pro Phe Pro His His Arg Tyr Ala Leu Val Asp Met Ala Asn Ala Val
   805 810 815
   cgg cca ctg agt tgg ttc tag
   2469
   Arg Pro Leu Ser Trp Phe
   820
<210> 4
   <211> 822
   <212> PRT
   <213> Corynebacterium glutamicum
<400> 4
   Met Lys Asp Asn Glu Asp Phe Asp Pro Asp Ser Pro Ala Thr Glu Ala
   1 5 10 15
   Val Ala Phe Asn Pro Phe Asp Asp Asp Asp Glu Asp Asp Ser Pro Ala
   20 25 30
   Thr Ser Ala Val Ala Phe Asn Pro Phe Glu Asp Asp Asp Asp Asp Asp
   35 40 45
   Glu Phe Gln Gly Glu Gly Leu Glu Phe Leu Leu Arg Asp Leu Asp Asn
   50 55 60
   Leu Arg Ala Thr Gln Gly Gln Met Val Val Glu Gln Pro Ala Val Glu
   65 70 75 80
   Asp Ser Leu Gly Ser Ala Ser Ala His Thr Glu Thr Thr Ala Ala Ser
   85 90 95
   Leu Arg Pro Arg Pro Glu Val Asp Pro Ser Glu Arg Ser Arg Arg Gln
   100 105 110
   Ala Ile Ser Leu Phe Arg Glu Arg Arg Arg Val Arg Arg Gln Ser Arg
   115 120 125
   Pro Val Ala Asp Gly Met Val Glu Leu Pro Phe Ile Thr Pro Lys Pro
   130 135 140
   Glu Asp Glu Leu Leu Ile Asp Pro Glu Lys Lys Arg Lys Pro Gly Val
   145 150 155 160
   Ala Ala Pro Gln Leu Val Ala Gly Asp Ile Val Ala Glu Gln Tyr Glu
   165 170 175
   Val Leu Gly Val Ile Ala His Gly Gly Met Gly Trp Ile Tyr Leu Ala
   180 185 190
   Asn Asp Arg Asn Val Ser Gly Arg Ile Val Val Leu Lys Gly Met Met
   195 200 205
   Ala Gln Ser Ser Val Gln Asp Gln Gly Thr Ala Glu Ala Glu Arg Glu
   210 215 220
   Phe Leu Ala Asp Ile Thr His Pro Gly Ile Val Lys Ala Tyr Asn Phe
   225 230 235 240
   Ile Asp Asp Pro Arg Val Pro Gly Gly Phe Ile Val Met Glu Tyr Val
   245 250 255
   Asn Gly Pro Ser Leu Lys Asp Arg Cys Lys Ala Gln Pro Asp Gly Val
   260 265 270
   Leu Arg Val Asp Leu Ala Ile Gly Tyr Ile Leu Glu Leu Leu Pro Ala
   275 280 285
   Met Asp Tyr Leu His Gln Arg Gly Val Val Tyr Asn Asp Leu Lys Pro
   290 295 300
   Glu Asn Val Ile Ala Thr Glu Asp Gln Val Lys Leu Ile Asp Leu Gly
   305 310 315 320
   Ala Val Thr Gly Ile Gly Ala Phe Gly Tyr Ile Tyr Gly Thr Lys Gly
   325 330 335
   Phe Gln Ala Pro Glu Val Ala Thr His Gly Pro Ser Ile Ser Ser Asp
   340 345 350
   Ile Phe Thr Ile Gly Arg Thr Leu Ala Ala Leu Thr Met Pro Leu Pro
   355 360 365
   Val Glu Asp Gly Val Leu Ala Pro Gly Ile Pro Ser Pro Lys Asn Ser
   370 375 380
   Pro Leu Leu Arg Arg His Leu Ser Phe Tyr Arg Leu Leu Gln Arg Ala
   385 390 395 400
   Thr Ala Asp Asp Pro Gln His Arg Phe Arg Asn Val Ser Glu Leu Arg
   405 410 415
   Thr Gln Leu Tyr Gly Val Leu Arg Glu Ile Leu Ala Val Arg Asp Gly
   420 425 430
   Lys Gln Tyr Pro Pro Gln His Ser Leu Phe Ser Pro Gln Arg Ser Thr
   435 440 445
   Phe Gly Thr Lys His Leu Val Phe Arg Thr Asp Arg Ile Ile Asp Gly
       450                 455                 460
   Ile Glu Arg Gln Ala Arg Ile Thr Ala Pro Glu Ile Val Ser Ala Leu
   465                 470                 475                 480
   Pro Val Pro Leu Ile Asp Arg Thr Asp Pro Gly Ala Arg Met Leu Ser
                   485                 490                 495
   Gly Ser Ser Tyr Ala Glu Pro Ser Glu Thr Leu Glu Thr Leu Arg Asn
               500                 505                 510
   Ser Met Glu Asp Glu Gln Tyr Arg Gln Ser Ile Glu Ile Pro Leu Gly
           515                 520                 525
   Val Val Arg Ala Leu Leu Asp Leu Gly Phe Thr Thr Glu Ala Arg Gln
       530                 535                 540
   Trp Leu Glu Thr Leu Glu Gly Arg Ile Gly Asp Asp Trp Arg His Lys
   545                 550                 555                 560
   Trp Phe Ser Gly Ile Thr Tyr Leu Leu Leu Asp Asp Tyr Ala Thr Ala
                   565                 570                 575
   Gln Val Phe Phe Asn His Val Leu Thr Ile Leu Pro Gly Glu Ala Ala
               580                 585                 590
   Pro Lys Leu Ala Leu Ala Ala Val Asp Glu Leu Ile Leu Gln Gln Ile
           595                 600                 605
   Gly Ala Glu Ser Thr Ala Tyr Leu Thr Pro Asp Ile Val Ser Ala Thr
       610                 615                 620
   Ala Thr Leu Ser Lys Asp Phe Glu Asp Leu Asp Ala Ser Ala Phe Glu
   625 630 635 640
   Ser Leu Ser Asp Thr Trp Ser His Ile Ser Ser Asp Pro His Val Val
   645 650 655
   Arg Phe His Ser Leu Arg Leu Tyr Ala Leu Val Trp Ala Thr Asn Pro
   660 665 670
   Thr Thr Val Ser Ser Ala Phe Gly Leu Ala Arg Gln Leu Met Ala Glu
   675 680 685
   Asn Gln Ile Glu Leu Ala Val Gln Ala Leu Asp Lys Leu Pro Gln Ser
   690 695 700
   Ser Thr His Tyr Arg Met Ala Thr Leu Thr Thr Ile Leu Leu Leu Val
   705 710 715 720
   Ser Ser Asn Leu Ser Glu Ser Arg Ile Arg Arg Ala Ala Arg Arg Leu
   725 730 735
   Thr Glu Ile Pro Thr Asn Glu Pro Arg Phe Asn Gln Ile Lys Ile Ala
   740 745 750
   Ile Met Ser Ala Gly Leu Ser Trp Leu Arg Glu Arg Lys Leu Lys Ala
   755 760 765
   Ser Ala Ser Ala Asn Pro Leu Phe Glu Tyr Pro Phe Ser Gln Lys Gly
   770 775 780
   Leu Arg Thr Gly Ile Ser Glu Ala Leu Arg Ile Gln Ala Arg Ser Ala
   785 790 795 800
   Pro Phe Pro His His Arg Tyr Ala Leu Val Asp Met Ala Asn Ala Val
   805 810 815
   Arg Pro Leu Ser Trp Phe
   820
<210> 5
   <211> 27
   <212> DNA
   <213> Artificial
<220>
   <223> primer
<400> 5
   gacgtcgacg atcaccgacg aacgcgc
   27
<210> 6
   <211> 46
   <212> DNA
   <213> Artificial
<220>
   <223> primer
<400> 6
   cccatccact aaacttaaac aatcttcatt atccttcatc gttttc
   46
<210> 7
   <211> 41
   <212> DNA
   <213> Artificial
<220>
   <223> primer
<400> 7
   tgtttaagtt tagtggatgg ggcgaatgcc gtgcggccac t
   41
<210> 8
   <211> 29
   <212> DNA
   <213> Artificial
<220>
   <223> primer
<400> 8
   gattctagag ttagctatcg ctaggtacg
   29
<210> 9
   <211> 20
   <212> DNA
   <213> Artificial
<220>
   <223> primer
<400> 9
   gctgcgcggt tttgaagtgg
   20
<210> 10
   <211> 21
   <212> DNA
   <213> Artificial
<220>
   <223> primer
<400> 10
   gatcgatcca gagcgtaacg c
   21
<210> 11
   <211> 28
   <212> DNA
   <213> Artificial
<220>
   <223> primer
<400> 11
   gacaagcttg atcagtccgt gagggaac
   28
<210> 12
   <211> 44
   <212> DNA
   <213> Artificial
<220>
   <223> primer
<400> 12
   cccatccact aaacttaaac agccgttgtt gtcgctcatt aaac
   44
<210> 13
   <211> 43
   <212> DNA
   <213> Artificial
<220>
   <223> primer
<400> 13
   tgtttaagtt tagtggatgg gaagttccgc ctggttttcc tcg
   43
<210> 14
   <211> 27
   <212> DNA
   <213> Artificial
<220>
   <223> primer
<400> 14
   gtcggatccg aggaggaacc aaggatg
   27
<210> 15
   <211> 27
   <212> DNA
   <213> Artificial
<220>
   <223> primer
<400> 15
   gacggatcct gagaactatg aaattcc
   27
<210> 16
   <211> 51
   <212> DNA
   <213> Artificial
<220>
   <223> primer
<400> 16
   gaggatcctt acttctcgaa ctgtgggtgg gaccactcag cagggcctgc g
   51
<210> 17
   <211> 29
   <212> DNA
   <213> Artificial
<220>
   <223> primer
<400> 17
   gccacaggtc gaggccacct cagtattcc
   29
<210> 18
   <211> 29
   <212> DNA
   <213> Artificial
<220>
   <223> primer
<400> 18
   ggaatactga ggtggcctcg acctgtggc
   29